# EUROPÄISCHE PATENTANMELDUNG

(11) **EP 4 843 505 A1**
(43) Veröffentlichungstag der Anmeldung: **25.11.2026**
(21) Anmeldenummer: 25177951.8
(22) Anmeldetag: 21.05.2025
(51) Int. Cl.: A61B 6/02, A61B 6/06, A61B 6/40, A61B 6/50

(54) **VERFAHREN ZUM BELEUCHTEN EINES RÖNTGENSTRAHLUNGSFELDES**

(71) Anmelder: Siemens Healthineers AG, 91301 Forchheim (DE)
(72) Erfinder: FREUDENBERGER, Jörg, 90562 Kalchreuth (DE); HALL, Katrin, 91330 Eggolsheim (DE); KAPPLER, Steffen, 91352 Hallerndorf-Pautzfeld (DE); NANKE, Ralf, 91077 Neunkirchen am Brand (DE); RADICKE, Marcus, 90587 Veitsbronn (DE)
(74) Vertreter: Siemens Healthineers Patent Attorneys

(57) **Zusammenfassung**

Die Erfindung betrifft ein computerimplementiertes Verfahren zum Beleuchten eines Röntgenstrahlungsfeldes (200). In dem Verfahren wird eine Röntgenquelle (600) relativ zu einem Röntgendetektor (505) während eines ersten und eines zweiten Belichtungszeitraums (T1, T2) bewegt (MOV-S), wobei der Röntgendetektor (505) die von der Röntgenquelle (600) ausgesendete Röntgenstrahlung (R) während der Belichtungszeiträume (T1, T2) empfängt (ACQ1, ACQ2), und zwischen der Röntgenquelle (600) und dem Röntgendetektor (505) eine Kollimatoreinheit (300, 300', 300", 300‴, 300ʺʺ, 300‴ʺ) angeordnet ist, die die Röntgenstrahlung (R) auf das Röntgenstrahlungsfeld (200) begrenzt. Zudem wird der Fokus (630) der Röntgenstrahlung (R) während des ersten und des zweiten Belichtungszeitraums relativ zur Bewegung der Röntgenquelle (600) bewegt (MOV-F1, MOV-F2). Dabei wird die Kollimatoreinheit (300, 300', 300", 300‴, 300"", 300‴ʺ) derart bewegt (MOV-C1, MOV-C2), dass das Röntgenstrahlungsfeld (200) konstant beleuchtet wird. Die Kollimatoreinheit (300, 300', 300", 300‴, 300"", 300‴ʺ) nimmt zu Beginn Belichtungszeiträume (T1, T2) verschiedene Startpositionen (SPOS1, SPOS2) ein.

## Beschreibung

Die vorliegende Offenbarung betrifft ein computerimplementiertes Verfahren, eine Kollimatoreinheit, ein Steuersystem, ein Mammographiesystem, ein Computerprogrammprodukt und ein computerlesbares Speichermedium zum Beleuchten eines Röntgenstrahlungsfeldes.

Die vorliegende Erfindung betrifft das Gebiet der medizinischen Bildgebung, insbesondere der Mammographie und Tomosynthese.

In der röntgenbasierten medizinischen Bildgebung wird typischerweise ein Untersuchungsobjekt zwischen einer Röntgenquelle und einem Röntgendetektor angeordnet. Es ist bekannt, insbesondere für dreidimensionale Aufnahmen und/oder zur Bestimmung von Schichtbildern des Untersuchungsobjektes, die Röntgenquelle relativ zu dem Untersuchungsobjekt und dem Röntgendetektor zu bewegen. Das Untersuchungsobjekt ist dabei insbesondere ein Mensch bzw. ein Patient.

Insbesondere für eine Tomosynthese in der Mammographie wird die Röntgenquelle auf einer bogenförmigen Bahn relativ zu dem Untersuchungsobjekt bewegt. Während mehrerer Belichtungszeiträumen wird dabei von der Röntgenquelle ausgesendete Röntgenstrahlung aus verschiedenen Winkeln von dem Röntgendetektor erfasst und als mehrere Bilddaten des Untersuchungsobjektes bereitgestellt. Es ist bekannt, mit einem Kollimator einen von der Röntgenstrahlung beleuchteten Bereich, das Röntgenstrahlungsfeld, auf dem Untersuchungsobjekt bzw. dem Röntgendetektor zu begrenzen.

Es ist bekannt, dass ein Fokus der Röntgenstrahlung auf einer Anode der Röntgenquelle während eines Belichtungszeitraums entgegengesetzt zu der Bewegung der Röntgenquelle bewegt werden kann, um ein Verschmieren des Fokus' aus Sicht des Röntgendetektors zu reduzieren. Außerdem kann der Fokus der Röntgenstrahlung während eines Belichtungszeitraums bewegt werden, um eine bestimmte Fokusform aus Sicht des Röntgendetektors zu erhalten.

Durch die Bewegung der Röntgenquelle und durch die Bewegung des Fokus' ist das Röntgenstrahlungsfeld typischerweise während eines Belichtungszeitraums nicht ortsfest. Dies kann dazu führen, dass das Röntgenstrahlungsfeld über einen Rand des Röntgendetektors hinaus geht und somit auch Gegenstände oder Teile des Untersuchungsobjektes, welche hinter dem Röntgendetektor angeordnet sind, mit Röntgenstrahlung beleuchtet werden. Außerdem werden durch das nicht ortsfeste Röntgenstrahlungsfeld unter Umständen Bereiche des Untersuchungsobjektes mit Röntgenstrahlung beleuchtet, die für das Erfassen der Bilddaten nicht notwendigerweise beleuchtet werden müssen. Außerdem kann durch das instabile, nichts ortsfeste Röntgenstrahlungsfeld eine Bildqualität der bereitgestellten Bilddaten reduziert werden, da in verschiedenen Bilddaten verschiedene Bereiche des Untersuchungsobjektes abgebildet sind und unter Umständen eine dreidimensionale Rekonstruktion der Bilddaten erschwert wird.

Das zu lösende objektive Problem besteht somit darin, ein Verfahren und eine Vorrichtung bereitzustellen, mit welchen das Röntgenstrahlungsfeld konstant, insbesondere ortsfest gehalten werden kann.

Dieses objektive Problem wird durch ein computerimplementiertes Verfahren, eine Kollimatoreinheit, ein Steuersystem, ein Mammographiesystem, ein Computerprogrammprodukt und ein computerlesbares Speichermedium gemäß den unabhängigen Ansprüchen gelöst. Vorteilhafte Merkmale und Weiterentwicklungen sind in den abhängigen Ansprüchen und in der folgenden Beschreibung aufgeführt.

Unabhängig von der verwendeten grammatikalischen Form sind Personen männlichen, weiblichen oder anderen Geschlechts in dem Begriff eingeschlossen.

Im Folgenden wird die erfindungsgemäße Lösung sowohl in Bezug auf die beanspruchten Systeme als auch in Bezug auf die beanspruchten Verfahren beschrieben. Merkmale, Vorteile oder alternative Ausführungsformen hierin können den anderen beanspruchten Gegenständen zugeordnet werden und umgekehrt. Mit anderen Worten können Ansprüche für die Systeme mit Merkmalen verbessert werden, die im Zusammenhang mit den Verfahren beschrieben oder beansprucht werden. In diesem Fall werden die funktionalen Merkmale des Verfahrens durch objektive Einheiten des Systems verkörpert.

Die Erfindung betrifft in einem ersten Aspekt ein computerimplementiertes Verfahren zum Beleuchten eines Röntgenstrahlungsfeldes. In einem Verfahrensschritt eines Bewegens einer Röntgenquelle wird eine Röntgenquelle relativ zu einem Röntgendetektor während eines ersten und eines zweiten Belichtungszeitraums bewegt. Der Röntgendetektor ist dazu ausgebildet, von der Röntgenquelle ausgesendete Röntgenstrahlung während des ersten und des zweiten Belichtungszeitraums zu empfangen. Zwischen der Röntgenquelle und dem Röntgendetektor ist eine Kollimatoreinheit angeordnet. Die Kollimatoreinheit ist dazu ausgebildet, die Röntgenstrahlung auf das Röntgenstrahlungsfeld zu begrenzen. In einem weiteren Verfahrensschritt eines Bewegens eines Fokus' während des ersten Belichtungszeitraums wird der Fokus der Röntgenstrahlung relativ zu der Bewegung der Röntgenquelle während des ersten Belichtungszeitraums bewegt. In einem weiteren Verfahrensschritt eines Bewegens der Kollimatoreinheit während des ersten Belichtungszeitraums wird die Kollimatoreinheit derart bewegt, dass während des ersten Belichtungszeitraums das Röntgenstrahlungsfeld konstant beleuchtet wird. In einem weiteren Verfahrensschritt eines Bewegens des Fokus' während des zweiten Belichtungszeitraums wird der Fokus der Röntgenstrahlung relativ zu der Bewegung der Röntgenquelle während des zweiten Belichtungszeitraums bewegt. In einem weiteren Verfahrensschritt eines Bewegens der Kollimatoreinheit während des zweiten Belichtungszeitraums wird die Kollimatoreinheit derart bewegt, dass während des zweiten Belichtungszeitraums das Röntgenstrahlungsfeld konstant beleuchtet wird. Dabei nimmt die Kollimatoreinheit zu Beginn des ersten Belichtungszeitraums eine erste Startposition ein und zu Beginn des zweiten Belichtungszeitraums eine zweite Startposition ein. Die erste und die zweite Startposition sind verschieden.

Ein computerimplementiertes Verfahren kann als eine Abfolge von Schritten bzw. Verfahrensschritten verstanden werden, die von einem Computer oder einer anderen programmierbaren Vorrichtung ausgeführt werden. Das Verfahren kann in Form von Computerprogrammcode oder Softwareanweisungen implementiert sein, die von einem Prozessor ausgeführt werden.

In dem Verfahrensschritt des Bewegens der Röntgenquelle wird die Röntgenquelle während des ersten und des zweiten Belichtungszeitraums relativ zu dem Röntgendetektor bewegt. Insbesondere kann die Röntgenquelle während des gesamten ersten und des gesamten zweiten Belichtungszeitraums bewegt werden. Alternativ kann die Röntgenquelle lediglich während eines Teils des ersten und/oder lediglich während eines Teils des zweiten Belichtungszeitraums bewegt werden. Insbesondere kann die Röntgenquelle zwischen den Belichtungszeiträumen weiterbewegt werden. Alternativ kann die Röntgenquelle zwischen dem ersten und dem zweiten Belichtungszeitraum anhalten bzw. pausieren.

Der Röntgendetektor ist dabei derart ausgebildet, dass er von der Röntgenquelle ausgesendete Röntgenstrahlung während des ersten und des zweiten Belichtungszeitraums empfängt.

Insbesondere empfängt der Röntgendetektor einen Teil der von der Röntgenquelle ausgesendeten Röntgenstrahlung.

Die Röntgenquelle ist als eine Vorrichtung ausgebildet, die Röntgenstrahlung erzeugt. In der Röntgenquelle können Elektronen auf eine Anode beschleunigt werden. Durch das Abbremsen der Elektronen in dem Anodenmaterial und durch die Anregung des Anodenmaterials wird Röntgenstrahlung zu erzeugen. Die Röntgenquelle kann beispielsweise als Drehanoden-Röntgenquelle ausgebildet sein. Typischerweise wird der Elektronenstrahl in der Röntgenquelle auf einen Bereich auf der Anode fokussiert.

Der Röntgendetektor ist als eine Vorrichtung ausgebildet, die Röntgenstrahlung empfängt und in ein elektrisches Signal umwandelt. Der Röntgendetektor kann beispielsweise aus einem Szintillator bestehen, der die Röntgenstrahlung in sichtbares Licht umwandelt, welches dann von Photodioden erfasst wird. Alternativ kann der Röntgendetektor einen Halbleiter zum Empfangen der Röntgenstrahlung umfassen.

Insbesondere kann zwischen der Röntgenquelle und dem Röntgendetektor ein Untersuchungsobjekt angeordnet sein. Das Untersuchungsobjekt kann dabei insbesondere ein Mensch bzw. ein Patient oder ein Tier oder ein Gegenstand sein. Insbesondere kann das Untersuchungsobjekt eine menschliche Brust sein. Alternativ kann das Untersuchungsobjekt auch ein Phantom oder ein anderes Testobjekt sein, das für Kalibrierungszwecke oder zur Qualitätssicherung verwendet wird.

Der erste und der zweite Belichtungszeitraum kann jeweils als der Zeitraum definiert sein, in dem der Röntgendetektor von der Röntgenquelle ausgesendete Röntgenstrahlung erfasst bzw. empfängt. Insbesondere kann die in einem Belichtungszeitraum erfasste Röntgenstrahlung in dem Röntgendetektor über den gesamten Belichtungszeitraum integriert werden. Der Röntgendetektor kann diese Röntgenstrahlung als Bilddaten bereitstellen.

Der erste und der zweite Belichtungszeitraum können zeitlich direkt aneinander anschließen. Alternativ können der erste und der zweite Belichtungszeitraum zeitlich durch eine Pause getrennt sein. Der erste und der zweite Belichtungszeitraum können gleich oder verschieden lang sein.

Das Bewegen der Röntgenquelle relativ zum Röntgendetektor kann bedeuten, dass entweder die Röntgenquelle, der Röntgendetektor oder beide bewegt werden, sodass sich ihre relative Position zueinander ändert. Diese Bewegung kann beispielsweise eine Rotation, Translation oder eine Kombination aus beidem sein.

Die Bewegung der Röntgenquelle relativ zum Röntgendetektor kann eine kontinuierliche oder schrittweise Positionsänderung der Röntgenquelle in Bezug auf den ortsfesten Röntgendetektor umfassen.

Die Bewegung der Röntgenquelle kann insbesondere als eine bogenförmige Bewegung relativ zu dem Röntgendetektor ausgebildet sein. Hierbei kann sich die Röntgenquelle auf einem Kreisbogen um einen Mittelpunkt bewegen, wobei der Röntgendetektor ortsfest bleibt. Die Bewegung der Röntgenquelle kann kontinuierlich erfolgen, das heißt ohne Unterbrechungen oder Haltepunkte während des Belichtungszeitraums. In Ausführungen der Erfindung, kann der Röntgendetektor mit der Röntgenquelle mitkippen, sodass eine Oberfläche des Röntgendetektors der Röntgenquelle zugewandt bleibt, während diese um den Röntgendetektor herum rotiert bzw. dreht bzw. schwenkt.

Zwischen der Röntgenquelle und dem Röntgendetektor ist die Kollimatoreinheit angeordnet. Die Kollimatoreinheit ist dabei derart ausgebildet, dass sie die Röntgenstrahlung auf das Röntgenstrahlungsfeld begrenzt.

Die Kollimatoreinheit ist als eine Vorrichtung ausgebildet, die den Röntgenstrahl formt und begrenzt. Die Kollimatoreinheit kann beispielsweise Stahllamellen, Bleilamellen oder Wolframlamellen oder andere strahlungsabsorbierenden Materialien umfassen, die so angeordnet sind, dass sie nur Strahlung in einem bestimmten Winkel oder Bereich durchlassen.

Das Begrenzen der Röntgenstrahlung auf das Röntgenstrahlungsfeld durch die Kollimatoreinheit kann bedeuten, dass die Kollimatoreinheit so eingestellt und/oder bewegt wird, dass sie nur Röntgenstrahlung in dem gewünschten Bereich (dem Röntgenstrahlungsfeld) durchlässt und Strahlung außerhalb dieses Bereichs blockiert.

In dem Verfahrensschritt des Bewegens des Fokus' der Röntgenstrahlung während des ersten Belichtungszeitraums und in dem Verfahrensschritt des Bewegens des Fokus' der Röntgenstrahlung während des zweiten Belichtungszeitraums wird der Fokus der Röntgenstrahlung relativ zu der Bewegung der Röntgenquelle während des ersten bzw. zweiten Belichtungszeitraums bewegt.

Der erste und der zweite Belichtungszeitraum können aufeinanderfolgende oder getrennte Zeiträume sein, in denen die Röntgenquelle aktiviert ist und Röntgenstrahlung emittiert und die Röntgenstrahlung vom Röntgendetektor erfasst wird. Diese Zeiträume können optional in ihrer Dauer variieren und können optional durch Pausen getrennt sein, in denen keine Strahlung emittiert wird.

Der Fokus der Röntgenstrahlung kann als der Punkt oder Bereich definiert sein, von dem die Röntgenstrahlung auszugehen scheint. Bei der Röntgenröhre kann dies der Punkt bzw. der Bereich auf der Anode sein, auf den der Elektronenstrahl trifft und in welchem die Röntgenstrahlung generiert wird.

Das Bewegen des Fokus' relativ zur Bewegung der Röntgenquelle kann bedeuten, dass der Punkt, von dem die Röntgenstrahlung ausgeht, innerhalb der Röntgenquelle verschoben wird, während sich die gesamte Röntgenquelle bewegt. Die Bewegung des Fokus' kann dabei durch verschiedene Mechanismen realisiert werden, beispielsweise durch eine elektrostatische oder eine magnetische Ablenkung des Elektronenstrahls in der Röntgenquelle oder durch eine mechanische Verschiebung der Anode. Insbesondere kann der Fokus mittels einer Ablenkeinheit bewegt werden. Mögliche Ausführungsformen einer Ablenkeinheit umfassen Elektromagnete, elektrostatische Ablenkplatten oder Spulen. Die Ablenkeinheit kann innerhalb der Röntgenquelle, insbesondere innerhalb eines Eintanks der Röntgenquelle oder innerhalb eines Vakuumgehäuses der Röntgenquelle angeordnet sein.

Dabei wird in den Verfahrensschritten des Bewegens der Kollimatoreinheit während des ersten Belichtungszeitraums bzw. während des zweiten Belichtungszeitraums die Kollimatoreinheit so bewegt, dass während des ersten Belichtungszeitraums bzw. während des zweiten Belichtungszeitraums das Röntgenstrahlungsfeld konstant beleuchtet wird.

Dabei nimmt die Kollimatoreinheit zu Beginn des ersten Belichtungszeitraums eine erste Startposition ein und zu Beginn des zweiten Belichtungszeitraums eine zweite Startposition ein, wobei die erste und die zweite Startposition verschieden sind.

Dies kann durch ein Steuersystem realisiert werden, das die Position der Kollimatoreinheit basierend auf der Position der Röntgenquelle und des Fokus berechnet und entsprechend anpasst.

Das konstante Beleuchten des Röntgenstrahlungsfeldes bedeutet, dass trotz der Bewegung der Röntgenquelle und des Fokus' das Röntgenstrahlungsfeld relativ zu dem Untersuchungsobjekt oder dem Röntgendetektor ortsfest bleibt. Insbesondere kann dabei die Intensität und/oder Verteilung der Röntgenstrahlung im definierten Röntgenstrahlungsfeld gleichmäßig bleiben. Konstant bedeutet in diesem Zusammenhang insbesondere "im Wesentlichen konstant". Kleinere Schwankungen oder Abweichungen sollen von dem Wortlaut umfasst sein.

Die Bewegung der Kollimatoreinheit kann dabei durch einen oder mehrere Motoren realisiert werden.

Die Bewegung der Kollimatoreinheit kann als eine Verschiebung, Rotation oder Kombination aus beidem ausgebildet sein. Die Bewegung kann kontinuierlich oder schrittweise erfolgen.

Die Bewegung der Kollimatoreinheit erfolgt dabei insbesondere in Abhängigkeit bzw. korreliert mit der Bewegung des Fokus in dem jeweiligen Belichtungszeitraum.

Die erste bzw. zweite Startposition der Kollimatoreinheit kann als die anfängliche Ausrichtung oder Position der Kollimatoreinheit zu Beginn eines Belichtungszeitraums definiert sein. Diese Position kann beispielsweise durch eine Stellung von von der Kollimatoreinheit umfassten Lamellen oder anderer Komponenten der Kollimatoreinheit charakterisiert sein. Insbesondere kann die erste Startposition von einer Position der Röntgenquelle relativ zu dem Röntgendetektor zu Beginn des ersten Belichtungszeitraums abhängen. Insbesondere kann die zweite Startposition von einer Position der Röntgenquelle relativ zu dem Röntgendetektor zu Beginn des zweiten Belichtungszeitraums abhängen.

Die Bewegung der Röntgenquelle, des Fokus' und der Kollimatoreinheit kann durch ein zentrales Steuersystem koordiniert werden. Dieses Steuersystem kann beispielsweise einen Mikroprozessor, einen Mikrocontroller oder einen speziell entwickelten ASIC (Application-Specific Integrated Circuit) umfassen. Alternativ können die Röntgenquelle, der Fokus und/oder die Kollimatoreinheit von separaten Steuersystemen bewegt werden.

Vorteilhafterweise kann durch dieses Verfahren eine gleichmäßige bzw. konstante Beleuchtung des Röntgenstrahlungsfeldes während der gesamten Belichtungszeit erreicht werden, was zu einer verbesserten Bildqualität führen kann. Zudem kann durch die angepasste Bewegung der Kollimatoreinheit die Strahlenbelastung für das Untersuchungsobjekt, insbesondere den Patienten reduziert werden, da die Röntgenstrahlung gezielter auf den relevanten Bereich fokussiert werden kann. Insbesondere kann durch das Verfahren eine Verschiebung des Röntgenstrahlungsfeldes während einer gesamten Aufnahme verhindert bzw. reduziert werden. Auf diese Weise kann verhindert werden, dass Bereiche des Untersuchungsobjektes mit der Röntgenstrahlung beleuchtet werden, die für die Bildgebung nicht relevant sind. Auf diese Weise kann eine Strahlenbelastung für das Untersuchungsobjekt auf das notwendige Maß reduziert werden.

In einer optionalen Ausführungsform umfasst das Verfahren optional einen Verfahrensschritt eines Erfassens von ersten Bilddaten mit dem Röntgendetektor während des ersten Belichtungszeitraums. Das Verfahren umfasst dabei optional einen weiteren Verfahrensschritt eines Erfassens von zweiten Bilddaten mit dem Röntgendetektor während des zweiten Belichtungszeitraums. Das Verfahren umfasst optional einen weiteren Verfahrensschritt eines Bereitstellens der ersten und/oder zweiten Bilddaten.

Das Erfassen der ersten bzw. zweiten Bilddaten umfasst dabei insbesondere ein Erfassen der Röntgenstrahlung mit dem Röntgendetektor während des ersten bzw. zweiten Belichtungszeitraums. Der Röntgendetektor wandelt dabei die Röntgenstrahlung in ein elektrisches Signal um, welches als Bilddaten bereitgestellt werden kann.

Das Bereitstellen kann dabei insbesondere ein Bereitstellen mittels einer Schnittstelle umfassen. Insbesondere können die ersten und/oder zweiten Bilddaten einer Recheneinheit und/oder einer Speichereinheit bereitgestellt werden. Alternativ oder zusätzlich kann das Bereitstellen ein Anzeigen der ersten und/oder zweiten Bilddaten umfassen.

Insbesondere können die ersten Bilddaten eine Projektion des Untersuchungsobjektes aus einer ersten Position der Röntgenquelle auf den Röntgendetektor umfassen. Insbesondere können die zweiten Bilddaten eine Projektion des Untersuchungsobjektes aus einer zweiten Position der Röntgenquelle auf den Röntgendetektor umfassen.

In einer Ausführungsform des Verfahrens ist die Bewegung des Fokus' während des ersten und des zweiten Belichtungszeitraums derart ausgebildet, dass die Bewegung der Röntgenquelle relativ zum Röntgendetektor wenigstens teilweise derart kompensiert wird, dass der Fokus aus Sicht des Röntgendetektors ortsfest ist.

Die Kompensation der Bewegung der Röntgenquelle kann bedeuten, dass die Fokusbewegung der Bewegung der Röntgenquelle entgegenwirkt. Dies kann dazu führen, dass der Fokus aus der Perspektive des Röntgendetektors an einer festen Position zu verbleiben scheint, obwohl sich die Röntgenquelle bewegt.

Die Kompensation der Bewegung der Röntgenquelle kann vollständig oder teilweise erfolgen. Bei einer vollständigen Kompensation kann der Fokus aus Sicht des Röntgendetektors während des gesamten Belichtungszeitraums an derselben Position verbleiben. Bei einer teilweisen Kompensation kann sich der Fokus aus Sicht des Röntgendetektors während des Belichtungszeitraums geringfügig bewegen, wobei diese Bewegung jedoch deutlich geringer ausfallen kann als die Bewegung der Röntgenquelle. Insbesondere kann sich der Fokus bei einer teilweisen Kompensation während eines Teils des Belichtungszeitraums aus Sicht der Röntgendetektors bewegen und während eines anderen Teils des Belichtungszeitraums aus Sicht des Röntgendetektors an einer Position verbleiben bzw. statisch sein. Alternativ kann sich der Fokus bei einer teilweisen Kompensation während des Belichtungszeitraums oder während eines Teils des Belichtungszeitraums aus Sicht des Röntgendetektors langsamer bewegen als die Röntgenquelle.

Der Grad der Kompensation kann abhängig von den spezifischen Anforderungen der Bildgebung variieren. Insbesondere kann in Ausführungen der Erfindung eine Kompensation kürzer als der erste und/oder zweite Belichtungszeitraum erfolgen. Alternativ oder zusätzlich kann in Ausführungen der Erfindung die Kompensation derart ausgebildet sein, dass der Fokus nicht vollständig ortsfest ist, sich aber langsamer als die Röntgenquelle relativ zu dem Röntgendetektor bewegt. Vorteilhafterweise kann die Kompensation während des gesamten ersten und/oder zweiten Belichtungszeitraums erfolgen. Vorteilhafterweise ist die Bewegung des Fokus' genauso schnell, wie die Bewegung der Röntgenröhre in entgegengesetzte Richtung. Insbesondere kann in Ausführungen der Erfindung die Kompensation in dem ersten Belichtungszeitraum anders ausgebildet sein als die Kompensation in dem zweiten Belichtungszeitraum.

Durch die Kompensation der Bewegung der Röntgenquelle mittels der Bewegung des Fokus' kann eine verbesserte Bildqualität erzielt werden. Insbesondere kann eine Verwischung oder Unschärfe in den aufgenommenen Bilddaten bzw. Röntgenbildern reduziert werden, da der Fokus aus Sicht des Röntgendetektors ortsfest bleibt oder sich nur geringfügig bewegt. Dies kann insbesondere bei Aufnahmen mit langen Belichtungszeiten oder bei der Tomosynthese vorteilhaft sein.

In einer weiteren Ausführungsform des Verfahrens kann die Kollimatoreinheit während des ersten und/oder zweiten Belichtungszeitraums entgegengesetzt zu der Bewegung der Röntgenquelle bewegt werden.

Insbesondere wird die Kollimatoreinheit wenigstens während eines Teils des ersten und/oder zweiten Belichtungszeitraums entgegengesetzt zu der Bewegung der Röntgenquelle bewegt.

Entgegengesetzt zu der Bewegung der Röntgenquelle bedeutet, dass sich die Kollimatoreinheit in die entgegengesetzte Richtung zur Röntgenquelle bewegt. Wenn sich die Röntgenquelle beispielsweise nach rechts bewegt, kann sich die Kollimatoreinheit nach links bewegen. Die Bewegung der Kollimatoreinheit ist dabei so ausgeführt, dass sie der Bewegung der Röntgenquelle entgegenwirkt. Dies kann dazu beitragen, dass der Bereich des Röntgenstrahlungsfeldes, der von der Kollimatoreinheit begrenzt wird, relativ zum Untersuchungsobjekt oder zum Röntgendetektor konstant, insbesondere ortsfest, bleibt.

Die entgegengesetzte Bewegung der Kollimatoreinheit kann durch verschiedene Antriebsmechanismen realisiert werden. Beispielsweise kann ein Linearantrieb, ein Schrittmotor oder ein Servomotor verwendet werden, um die Kollimatoreinheit präzise zu bewegen. Die Steuerung dieser Bewegung kann durch das Steuersystem erfolgen, welches die Bewegung der Röntgenquelle und die Bewegung der Kollimatoreinheit koordiniert. Das Steuersystem kann insbesondere Positionssensoren und Antriebsmotoren umfassen, um die Bewegungen präzise zu steuern.

Die Geschwindigkeit und das Ausmaß der Bewegung der Kollimatoreinheit können an die Bewegung der Röntgenquelle und/oder an die Bewegung des Fokus' angepasst werden. Dies kann eine genaue Abstimmung zwischen der Bewegung der Röntgenquelle, der Bewegung des Fokus' und der Gegenbewegung der Kollimatoreinheit ermöglichen.

Durch die entgegengesetzte Bewegung der Kollimatoreinheit zur Röntgenquelle kann eine verbesserte Bildqualität erreicht werden. Die konstante Beleuchtung des Röntgenstrahlungsfeldes kann zu einer gleichmäßigeren Belichtung des Untersuchungsobjekts führen, was die Genauigkeit und Zuverlässigkeit der Bildgebung erhöhen kann. Vorteilhafterweise kann diese Ausführungsform eine effizientere Nutzung der Röntgenstrahlung ermöglichen. Durch die präzise Kontrolle des beleuchteten Röntgenstrahlungsfeldes kann die Strahlenbelastung für das Untersuchungsobjekt minimiert werden, während gleichzeitig die Bildqualität optimiert wird.

In einer weiteren Ausführungsform des Verfahrens kann das Röntgenstrahlungsfeld auf einer Oberfläche des Röntgendetektors angeordnet sein.

Das Röntgenstrahlungsfeld kann als der Bereich definiert werden, in dem die von der Röntgenquelle emittierte Röntgenstrahlung auf den Röntgendetektor trifft. Die Oberfläche des Röntgendetektors kann als die dem Untersuchungsobjekt bzw. der Röntgenquelle zugewandte Seite des Röntgendetektors verstanden werden, auf der die Röntgenstrahlung auftrifft und detektiert bzw. erfasst wird.

Das auf der Oberfläche des Röntgendetektors angeordnete Röntgenstrahlungsfeld kann in seiner Form und Größe variieren. Es kann beispielsweise rechteckig, quadratisch, kreisförmig oder einer anderen geometrischen Form entsprechend ausgebildet sein. Die Größe des Röntgenstrahlungsfeldes kann an die Größe des Röntgendetektors oder an die Größe des Untersuchungsobjekts angepasst sein. Insbesondere kann das Röntgenstrahlungsfeld die gesamte Oberfläche des Röntgendetektors umfassen und durch seine Kanten begrenzt werden. Alternativ kann das Röntgenstrahlungsfeld auf das Untersuchungsobjekt bzw. dessen interessierende Bereiche eingegrenzt sein.

Die Kollimatoreinheit kann in Ausführungen der Erfindung so konfiguriert sein, dass sie das Röntgenstrahlungsfeld genau auf die aktive Fläche des Röntgendetektors begrenzt.

Die Bewegung der Kollimatoreinheit kann so gesteuert werden, dass das Röntgenstrahlungsfeld während der Belichtungszeiträume stets optimal auf der Oberfläche des Röntgendetektors positioniert bleibt. Insbesondere kann das Röntgenstrahlungsfeld auf der Oberfläche des Röntgendetektors ortsfest sein. Dies kann eine gleichbleibende Bildqualität über den gesamten Aufnahmeprozess hinweg ermöglichen, selbst wenn sich die relative Position zwischen Röntgenquelle und Röntgendetektor ändert.

Vorteilhafterweise kann die Anordnung des Röntgenstrahlungsfeldes auf der Oberfläche des Röntgendetektors eine effiziente Nutzung der verfügbaren Röntgendetektorfläche ermöglichen. Dies kann besonders bei der Aufnahme von großflächigen Untersuchungsobjekten oder bei der Durchführung von Tomosynthese-Aufnahmen von Vorteil sein, bei denen eine möglichst vollständige Abdeckung des Untersuchungsbereichs erforderlich ist. Zusammenfassend kann die Anordnung des Röntgenstrahlungsfeldes auf der Oberfläche des Röntgendetektors zu einer verbesserten Bildqualität, einer erhöhten Detektionseffizienz und einer optimierten Nutzung der Röntgendetektorfläche beitragen. Dies kann insgesamt zu einer Verbesserung der diagnostischen Möglichkeiten und einer Reduzierung der Strahlenbelastung für das Untersuchungsobjekt führen.

In einer weiteren Ausführungsform des Verfahrens kann zwischen der Röntgenquelle und dem Röntgendetektor ein Untersuchungsobjekt angeordnet sein. Das Röntgenstrahlungsfeld kann in einer Ebene innerhalb des Untersuchungsobjektes angeordnet sein.

Das Untersuchungsobjekt kann dabei insbesondere wie oben beschrieben ausgebildet sein.

Das Röntgenstrahlungsfeld kann in einer Ebene innerhalb des Untersuchungsobjektes angeordnet sein. Die Ebene innerhalb des Untersuchungsobjektes kann als eine Fokusebene oder eine Schärfeebene bezeichnet werden. Diese Ebene kann parallel zur Oberfläche des Röntgendetektors ausgerichtet sein oder in einem bestimmten Winkel dazu stehen. Die Position dieser Ebene innerhalb des Untersuchungsobjektes kann basierend auf verschiedenen Faktoren gewählt werden, wie beispielsweise der Dicke des Untersuchungsobjektes, der Art der durchzuführenden Untersuchung oder spezifischen Bereichen von Interesse innerhalb des Untersuchungsobjektes. Beispielsweise kann die Ebene in der Mitte des Untersuchungsobjektes senkrecht zu einer Achse zwischen Röntgendetektor und Röntgenquelle angeordnet sein.

Die Anordnung des Röntgenstrahlungsfeldes in einer Ebene innerhalb des Untersuchungsobjektes kann ermöglichen, dass bestimmte Strukturen oder Bereiche innerhalb des Untersuchungsobjektes mit besonderer Schärfe oder Detailgenauigkeit abgebildet werden. Dies kann besonders vorteilhaft sein, wenn spezifische Regionen innerhalb des Untersuchungsobjektes von besonderem diagnostischem Interesse sind. Durch die Anordnung des Röntgenstrahlungsfeldes in einer Ebene innerhalb des Untersuchungsobjektes kann eine verbesserte Bildqualität in dieser spezifischen Ebene erreicht werden. Dies kann zu einer genaueren Diagnose oder Analyse der Strukturen in dieser Ebene führen.

In einer weiteren Ausführungsform des Verfahrens wird die Kollimatoreinheit basierend auf der Bewegung des Fokus' bewegt.

Mit anderen Worten kann die Bewegung der Kollimatoreinheit auf der Bewegung des Fokus' basieren bzw. von dieser abhängen. Die Bewegung des Fokus' kann als eine Eingangsgröße für die Steuerung der Bewegung der Kollimatoreinheit dienen. Das Basieren der Kollimatoreinheitbewegung auf der Fokusbewegung kann bedeuten, dass die Steuerung der Kollimatoreinheit die aktuelle Position und/oder Bewegungsrichtung des Fokus' berücksichtigt. Die Kollimatoreinheit kann so gesteuert werden, dass sie der Bewegung des Fokus' folgt.

Die Steuerung der Kollimatoreinheit basierend auf der Fokusbewegung kann in Echtzeit erfolgen, um eine kontinuierliche Anpassung der Kollimation während der Bildaufnahme zu ermöglichen. Alternativ kann die Bewegung der Kollimatoreinheit auch vorausberechnet und entsprechend einem vordefinierten Bewegungsprofil gesteuert werden.

Die Bewegung des Fokus' kann beispielsweise durch ein Steuersystem vorgegeben werden. Dieses Steuersystem kann auch dazu ausgebildet sein, die Bewegung der Kollimatoreinheit zu steuern. Dabei kann das Steuersystem die Bewegung der Kollimatoreinheit basierend auf der vorgegebenen oder gemessenen Bewegung des Fokus' berechnen und entsprechende Steuersignale an die Kollimatoreinheit senden.

Die Kopplung bzw. die Abhängigkeit zwischen Fokusbewegung und Kollimatoreinheitbewegung kann auf verschiedene Weise realisiert werden. In einer Ausführungsform kann ein gemeinsames Steuersignal sowohl für die Fokusbewegung als auch für die Bewegung der Kollimatoreinheit verwendet werden. Alternativ können separate, aber synchronisierte Steuersignale eingesetzt werden.

Die Bewegung der Kollimatoreinheit basierend auf der Fokusbewegung kann kontinuierlich oder in diskreten Schritten erfolgen. Bei einer kontinuierlichen Bewegung kann die Position der Kollimatoreinheit fortlaufend an die aktuelle Fokusposition angepasst werden. Bei einer schrittweisen Bewegung kann die Kollimatoreinheit in bestimmten Intervallen oder an definierten Positionen nachgeführt werden.

Durch die Kopplung der Bewegung der Kollimatoreinheit an die Fokusbewegung kann eine optimale Ausrichtung des Röntgenstrahlungsfeldes auf den aktuellen Fokus gewährleistet werden. Dies kann zu einer verbesserten Bildqualität führen, da Streustrahlung reduziert und die Beleuchtung des Röntgenstrahlungsfeldes optimiert werden kann. Vorteilhafterweise kann diese Ausführungsform eine effiziente Nutzung der Röntgenstrahlung ermöglichen, da die Kollimation präzise an die aktuelle Fokusposition angepasst wird. Dies kann zu einer Reduzierung der Strahlenbelastung für das Untersuchungsobjekt führen, da weniger Streustrahlung erzeugt wird. Zudem kann die Bildqualität verbessert werden und die Strahlenbelastung für das Untersuchungsobjekt minimiert werden, da das Röntgenstrahlungsfeld optimal und konstant beleuchtet wird.

In einer weiteren Ausführungsform des Verfahrens kann die Kollimatoreinheit außerdem basierend auf der Bewegung der Röntgenquelle bewegt werden.

Die Bewegung der Kollimatoreinheit kann zusätzlich zu der Bewegung basierend auf dem Fokus auch von der Bewegung der Röntgenquelle abhängen. Die Kollimatoreinheit kann so gesteuert werden, dass sie der Bewegung des Fokus' folgt und gleichzeitig die Bewegung der Röntgenquelle berücksichtigt.

Die Steuerung der Kollimatoreinheit kann beispielsweise durch ein Steuersystem erfolgen, das sowohl Informationen über die Fokusbewegung als auch über die Bewegung der Röntgenquelle erhält und verarbeitet. Basierend auf diesen Informationen kann das Steuersystem die Bewegung der Kollimatoreinheit so anpassen, dass stets eine optimale Kollimation gewährleistet ist.

Durch die Berücksichtigung beider Bewegungen - der Fokusbewegung und der Röntgenquellenbewegung - kann eine optimale Kollimation über den gesamten Belichtungszeitraum erreicht werden. Dies kann zu einer verbesserten Bildqualität und einer reduzierten Strahlenbelastung für das Untersuchungsobjekt führen. Insbesondere kann auf diese Weise die aus Sicht des Röntgendetektors aus der Überlagerung der Bewegung des Fokus' und der Bewegung der Röntgenquelle resultierende Bewegung derart kompensiert werden, dass das Röntgenstrahlungsfeld konstant, insbesondere ortsfest bleibt.

In einer weiteren Ausführungsform des Verfahrens kann die erste Startposition der Kollimatoreinheit von einer Position der Röntgenquelle relativ zum Röntgendetektor zu Beginn des ersten Belichtungszeitraums abhängen. Die zweite Startposition der Kollimatoreinheit kann von einer Position der Röntgenquelle relativ zum Röntgendetektor zu Beginn des zweiten Belichtungszeitraums abhängen.

Die Position der Röntgenquelle relativ zum Röntgendetektor kann dabei die räumliche Anordnung und Ausrichtung der Röntgenquelle in Bezug auf den Röntgendetektor zu einem bestimmten Zeitpunkt beschreiben. Diese relative Position kann beispielsweise durch Koordinaten in einem dreidimensionalen Raum oder durch Winkel angegeben werden. Beispielsweise kann die relative Position der Röntgenquelle zum Röntgendetektor durch den Abstand zwischen Röntgenquelle und Röntgendetektor und/oder den Winkel der Röntgenquelle bezüglich einer Referenzachse des Röntgendetektors beschrieben werden. Diese Parameter können durch geeignete Sensoren oder Positionierungssysteme erfasst werden.

Die erste Startposition der Kollimatoreinheit kann von einer Position der Röntgenquelle relativ zum Röntgendetektor zu Beginn des ersten Belichtungszeitraums abhängen. Der Beginn des ersten Belichtungszeitraums kann den Zeitpunkt markieren, an dem die Röntgenquelle aktiviert wird, um Röntgenstrahlung für die erste Aufnahme zu emittieren. Alternativ oder zusätzlich kann der Beginn des ersten Belichtungszeitraums den Zeitpunkt markieren, an dem der Röntgendetektor beginnt, Röntgenstrahlung für die ersten Bilddaten zu erfassen. Insbesondere kann zu diesem Zeitpunkt die Röntgenstrahlung bereits aktiviert sein. Zu diesem Zeitpunkt kann die Röntgenquelle eine bestimmte Position relativ zum Röntgendetektor einnehmen, die als Ausgangspunkt für die Bewegung während des Belichtungszeitraums dienen kann.

Die zweite Startposition der Kollimatoreinheit kann von einer Position der Röntgenquelle relativ zum Röntgendetektor zu Beginn des zweiten Belichtungszeitraums abhängen. Analog zum ersten Belichtungszeitraum kann der Beginn des zweiten Belichtungszeitraums den Zeitpunkt markieren, an dem die Röntgenquelle für die zweite Aufnahme aktiviert wird und/oder an dem der Röntgendetektor beginnt die Röntgenstrahlung für die zweiten Bilddaten zu erfassen. Insbesondere kann zu diesem Zeitpunkt die Röntgenstrahlung bereits aktiviert sein.

Die Abhängigkeit der ersten Startposition der Kollimatoreinheit von der Position der Röntgenquelle zu Beginn des ersten Belichtungszeitraums bzw. die Abhängigkeit der zweiten Startposition der Kollimatoreinheit von der Position der Röntgenquelle zu Beginn des zweiten Belichtungszeitraums kann bedeuten, dass die Kollimatoreinheit basierend auf dieser relativen Position ausgerichtet oder eingestellt wird. Dies kann beispielsweise umfassen, dass von der Kollimatoreinheit umfasste Lamellen so positioniert werden, dass sie das Röntgenstrahlungsfeld optimal auf den gewünschten Bereich begrenzen.

Die Abhängigkeit der ersten und zweiten Startposition der Kollimatoreinheit von der jeweiligen Position der Röntgenquelle kann durch ein Steuersystem realisiert werden. Dieses Steuersystem kann dazu ausgebildet sein, die aktuelle Position der Röntgenquelle zu erfassen und daraus die optimale Position der Kollimatoreinheit zu berechnen. Die Berechnung der optimalen ersten bzw. zweiten Startposition der Kollimatoreinheit kann verschiedene Faktoren berücksichtigen, wie beispielsweise die gewünschte Größe und Form des Röntgenstrahlungsfeldes, die Bewegung des Fokus oder die Geometrie des Untersuchungsobjekts. Basierend auf diesen Berechnungen kann das Steuersystem entsprechende Steuersignale an Aktuatoren der Kollimatoreinheit senden, um diese in die berechnete erste bzw. zweite Startposition zu bringen.

Die Position der Röntgenquelle zu Beginn des zweiten Belichtungszeitraums kann sich von der Position zu Beginn des ersten Belichtungszeitraums unterscheiden, da die Röntgenquelle zwischen den Aufnahmen bewegt worden sein kann. Entsprechend kann die Kollimatoreinheit für den zweiten Belichtungszeitraum in eine andere Startposition gebracht werden, um das Röntgenstrahlungsfeld weiterhin optimal zu begrenzen und insbesondere um sicherzustellen, dass in beiden Belichtungszeiträumen das gleiche Röntgenstrahlungsfeld beleuchtet wird.

Durch die Abhängigkeit der ersten bzw. zweiten Startposition der Kollimatoreinheit von der Position der Röntgenquelle kann eine präzise Ausrichtung des Röntgenstrahlungsfeldes auf den gewünschten Bereich des Untersuchungsobjekts bzw. des Röntgendetektors erreicht werden. Dies kann zu einer Verbesserung der Bildqualität und einer Reduzierung der Strahlenbelastung für das Untersuchungsobjekt führen. Insbesondere kann auf diese Weise auch bei einer variablen Positionierung der Röntgenquelle relativ zum Röntgendetektor das Röntgenstrahlungsfeld konstant, insbesondere ortsfest gehalten werden. Auf diese Weise kann ein Überstrahlen über die Ränder des Röntgendetektors und/oder ein unnötiges Bestrahlen von Bereichen des Untersuchungsobjektes, welche für die Bildgebung nicht relevant sind, vermieden werden. Vorteilhafterweise kann diese Ausführungsform eine dynamische Anpassung der Kollimation an verschiedene Aufnahmesituationen ermöglichen. Dies kann besonders nützlich sein bei Untersuchungsmethoden, bei denen die Röntgenquelle während der Aufnahme bewegt wird, wie beispielsweise bei der Tomosynthese.

In einer weiteren Ausführungsform des Verfahrens kann die Kollimatoreinheit wenigstens zwei Lamellen umfassen. Die erste Startposition kann wenigstens eine Startposition wenigstens einer der Lamellen relativ zu der Röntgenquelle zu Beginn des ersten Belichtungszeitraums vorgeben. Die zweite Startposition kann wenigstens eine Startposition wenigstens einer der Lamellen relativ zu der Röntgenquelle zu Beginn des zweiten Belichtungszeitraums vorgeben.

Die Kollimatoreinheit kann wenigstens zwei Lamellen umfassen. Die Lamellen können als bewegliche Elemente der Kollimatoreinheit ausgebildet sein, die dazu dienen, die Form und Größe des Röntgenstrahlungsfeldes zu definieren. Eine Lamelle kann als ein flaches oder gebogenes Element ausgebildet sein, das dazu konfiguriert ist, einen Teil der Röntgenstrahlung zu blockieren oder zu filtern. Die Lamellen können aus einem strahlungsabsorbierenden Material wie beispielsweise Stahl, Blei, Wolfram oder einer Bleilegierung hergestellt sein.

Eine Startposition einer Lamelle kann deren Position und/oder Orientierung relativ zur Röntgenquelle definieren. Diese Startposition kann durch verschiedene Parameter charakterisiert sein, wie beispielsweise den Abstand der Lamelle zur Röntgenquelle, den Winkel der Lamelle relativ zur Strahlungsachse oder die Ausrichtung der Lamelle im Raum.

Das Vorgeben einer Startposition kann das Einstellen oder Bewegen der entsprechenden Lamelle in die vorgegebene Position umfassen. Dies kann durch einen Antriebsmechanismus wie beispielsweise einen Elektromotor, einen Piezoantrieb oder einen hydraulischen Aktuator erfolgen.

Die Startpositionen der Lamellen zu Beginn des ersten und zweiten Belichtungszeitraums können sich unterscheiden, um die Kollimation an die jeweilige Position der Röntgenquelle und/oder des Fokus' anzupassen.

Die Vorgabe der Startpositionen der Lamellen kann durch ein Steuersystem erfolgen, das die Bewegungen der Kollimatoreinheit koordiniert. Das Steuersystem kann die Startpositionen basierend auf verschiedenen Faktoren berechnen, wie beispielsweise der geplanten Bewegung der Röntgenquelle, der geplanten Bewegung des Fokus', der gewünschten Größe und Form des Röntgenstrahlungsfeldes oder spezifischen Anforderungen des Untersuchungsprotokolls.

Vorteilhafterweise kann die Verwendung von wenigstens zwei Lamellen und die Möglichkeit, deren Startpositionen für verschiedene Belichtungszeiträume individuell vorzugeben, eine präzise und flexible Anpassung des Röntgenstrahlungsfeldes ermöglichen. Insbesondere kann auf diese Weise auf eine durch die geänderte Position der Röntgenquelle geänderte Geometrie des Röntgenstrahlungsfeldes eingegangen werden. Dies kann zu einer verbesserten Bildqualität und zu einer minimierten Strahlenbelastung für das Untersuchungsobjekt führen, indem die Strahlenbelastung auf die relevanten Bereiche des Untersuchungsobjekts begrenzt wird und gleichzeitig eine optimale Abdeckung des zu untersuchenden Bereichs sichergestellt wird.

In einer weiteren Ausführungsform des Verfahrens kann die Kollimatoreinheit wenigstens zwei Lamellen umfassen. Die erste Startposition kann wenigstens einen Abstand der beiden Lamellen zueinander zu Beginn des ersten Belichtungszeitraums vorgeben. Die zweite Startposition kann wenigstens einen Abstand der beiden Lamellen zueinander zu Beginn des zweiten Belichtungszeitraums vorgeben.

Die Lamellen können wie oben beschrieben ausgebildet sein.

Die erste Startposition kann wenigstens einen Abstand der beiden Lamellen zueinander zu Beginn des ersten Belichtungszeitraums vorgeben. Der Abstand zwischen den Lamellen kann die Größe und Form des Röntgenstrahlungsfeldes bestimmen. Zu Beginn des ersten Belichtungszeitraums können die Lamellen in einer definierten Ausgangsposition angeordnet sein, die durch die erste Startposition vorgegeben wird. Die zweite Startposition kann wenigstens einen Abstand der beiden Lamellen zueinander zu Beginn des zweiten Belichtungszeitraums vorgeben. Analog zur ersten Startposition kann die zweite Startposition eine definierte Ausgangsposition der Lamellen zu Beginn des zweiten Belichtungszeitraums festlegen.

Der Abstand zwischen den Lamellen in der ersten und zweiten Startposition kann unterschiedlich sein. Insbesondere kann der Abstand derart gewählt werden, dass zu Beginn jedes Belichtungszeitraums das Röntgenstrahlungsfeld identisch bzw. konstant bzw. ortsfest ist. Mit anderen Worten kann der Abstand an die Position der Röntgenquelle zu Beginn jedes Belichtungszeitraums derart angepasst werden, dass immer dasselbe Röntgenstrahlungsfeld ausgeleuchtet bzw. beleuchtet wird.

Die Vorgabe der Startpositionen kann durch ein Steuersystem erfolgen, das die Positionen der Lamellen kontrolliert. Die Startpositionen können basierend auf verschiedenen Faktoren wie der Position der Röntgenquelle, der Position des Fokus', der Größe des Untersuchungsobjekts oder der gewünschten Bildgebungsparameter bestimmt werden.

Die Einstellung des Abstands zwischen den Lamellen kann durch verschiedene Mechanismen erfolgen. Beispielsweise können die Lamellen an Linearverstelleinheiten angebracht sein, die eine präzise Positionierung ermöglichen. Die Steuerung dieser Linearverstelleinheiten kann durch ein computergestütztes System erfolgen, das die optimalen Abstände für jeden Belichtungszeitraum berechnet und einstellt.

Vorteilhafterweise kann diese Ausführungsform eine verbesserte Anpassung des Röntgenstrahlungsfeldes an die spezifischen Anforderungen verschiedener Belichtungszeiträume ermöglichen. Dies kann zu einer Optimierung der Bildqualität bei gleichzeitiger Minimierung der Strahlenbelastung für das Untersuchungsobjekt führen. Insbesondere kann durch die Anpassung des Abstandes zwischen den Lamellen sichergestellt werden, dass zu Beginn jedes Belichtungszeitraums das gleiche Röntgenstrahlungsfeld ausgeleuchtet bzw. beleuchtet wird und eine geänderte Geometrie durch eine veränderte Position der Röntgenquelle berücksichtigt wird. Auf diese Weise kann die Bildqualität verbessert und ein unnötiges Bestrahlen des Untersuchungsobjektes in nicht bildrelevanten Bereichen vermieden werden.

In einer weiteren Ausführungsform des Verfahrens kann die Bewegung der Röntgenquelle kontinuierlich sein. Insbesondere kann die Bewegung der Röntgenquelle als eine bogenförmige Bewegung relativ zu dem Röntgendetektor ausgebildet sein.

Eine kontinuierliche Bewegung kann als eine ununterbrochene, stetige Bewegung verstanden werden, bei der die Röntgenquelle ihre Position fortlaufend ändert, ohne anzuhalten oder abrupte Richtungswechsel vorzunehmen.

Eine bogenförmige Bewegung kann als eine Bewegung entlang eines Kreisbogens oder einer gekrümmten Bahn verstanden werden. Bei dieser Bewegungsform kann sich die Röntgenquelle auf einer gekrümmten Trajektorie um das Untersuchungsobjekt bewegen, wobei der Röntgendetektor als Bezugspunkt dient. Die Winkelabdeckung der bogenförmigen Bewegung kann dabei variabel sein und beispielsweise zwischen 15° und 180° betragen. Der Röntgendetektor kann dabei stationär bleiben oder sich ebenfalls bewegen, beispielsweise mit der Bewegung der Röntgenquelle mitkippen.

Eine kontinuierliche Bewegung der Röntgenquelle kann eine gleichmäßige und unterbrechungsfreie Aufnahme von Röntgenbildern ermöglichen. Dies kann zu einer verbesserten Bildqualität und einer reduzierten Strahlenbelastung für das Untersuchungsobjekt führen. Die bogenförmige Bewegung der Röntgenquelle relativ zu dem Röntgendetektor kann besonders vorteilhaft für tomographische Aufnahmen sein. Die bogenförmige Bewegung kann es ermöglichen, das Untersuchungsobjekt aus verschiedenen Winkeln zu durchleuchten. Dies kann zu einer verbesserten dreidimensionalen Darstellung des Untersuchungsobjekts führen. Insbesondere können die derart gleichmäßig erfassten Bilddaten leichter rekonstruiert werden, da die Positionen beim Erfassen der Bilddaten bekannt sind.

Die Erfindung betrifft in einem zweiten Aspekt eine Kollimatoreinheit, welche zur Verwendung in einem Verfahren nach einem der vorhergehenden Ausführungsformen ausgebildet ist.

Die Kollimatoreinheit kann dabei insbesondere wie oben beschrieben ausgebildet sein.

Die Kollimatoreinheit kann Blenden bzw. Lamellen umfassen, die beweglich ausgebildet sein können. Diese Blenden bzw. Lamellen können aus strahlungsabsorbierendem Material wie beispielsweise Stahl, Blei, Wolfram oder anderen Schwermetallen hergestellt sein. Die Blenden bzw. Lamellen können so angeordnet und konfiguriert sein, dass sie die Röntgenstrahlung auf einen definierten Bereich begrenzen.

Die Kollimatoreinheit kann wenigstens einen Antriebsmechanismus umfassen, der dazu ausgebildet ist, die Blenden bzw. Lamellen zu bewegen und zu positionieren. Dieser Antriebsmechanismus kann beispielsweise Elektromotoren, Piezoantriebe oder hydraulische Systeme umfassen.

Insbesondere kann die Kollimatoreinheit wenigstens ein Steuerelement aufweisen, das dazu konfiguriert ist, die Bewegung und Position der Blenden oder Lamellen zu kontrollieren. Dieses Steuerelement kann mit einem übergeordneten Steuersystem verbunden sein, um eine präzise Begrenzung des Röntgenstrahls auf das Röntgenstrahlungsfeld zu ermöglichen.

Die Kollimatoreinheit kann auch einen Filter oder ein anderes optisches Element umfassen, das dazu ausgebildet ist, die Eigenschaften des Röntgenstrahls zu modifizieren. Dieses kann beispielsweise dazu dienen, bestimmte Wellenlängen zu absorbieren oder die Intensität des Strahls anzupassen.

In einigen Ausführungsformen kann die Kollimatoreinheit als modulares System ausgebildet sein, das es ermöglicht, verschiedene Komponenten auszutauschen oder hinzuzufügen, um die Funktionalität an spezifische Anforderungen anzupassen.

Die Kollimatoreinheit kann so konfiguriert sein, dass sie in verschiedenen Röntgenbildgebungssystemen eingesetzt werden kann, einschließlich, aber nicht beschränkt auf, Mammographiesysteme, Computertomographen oder konventionelle Röntgengeräte.

Ferner kann die Kollimatoreinheit wenigstens einen Sensor umfassen, der dazu ausgebildet ist, die Position und Ausrichtung der Blenden bzw. Lamellen zu überwachen und Feedback an das Steuersystem zu liefern. Dies kann eine präzise und dynamische Anpassung der Strahlformung der Röntgenstrahlung auf das Röntgenstrahlungsfeld ermöglichen.

Ferner kann die Kollimatoreinheit wenigstens eine Abschirmung umfassen, die dazu ausgebildet ist, unerwünschte Streustrahlung zu reduzieren und den Strahlenschutz zu verbessern.

Die Kollimatoreinheit kann so ausgebildet sein, dass sie in Echtzeit auf Änderungen in der Röntgenquellenposition oder -ausrichtung reagieren kann, um eine konstante und präzise Strahlformung zu gewährleisten.

Die Kollimatoreinheit kann beweglich ausgebildet sein, um während eines Belichtungszeitraums bewegt zu werden. Die Kollimatoreinheit kann verschiedene Startpositionen zu Beginn verschiedener Belichtungszeiträume einnehmen. Die Bewegung der Kollimatoreinheit kann auf der Bewegung des Fokus' der Röntgenstrahlung und/oder der Bewegung der Röntgenquelle basieren.

Die Kollimatoreinheit kann in Ausführungen der Erfindung wenigstens zwei Lamellen umfassen, deren Positionen und/oder Abstände zueinander für verschiedene Belichtungszeiträume angepasst werden können.

Vorteilhafterweise kann durch die Verwendung einer solchen Kollimatoreinheit eine präzise und flexible Begrenzung des Röntgenstrahlungsfeldes erreicht werden. Insbesondere kann durch die Kollimatoreinheit sichergestellt werden, dass das Röntgenstrahlungsfeld konstant beleuchtet wird und insbesondere ortsfest bleibt. Dies kann zu einer verbesserten Bildqualität und einer reduzierten Strahlenbelastung für das Untersuchungsobjekt führen.

Folgende Ausführungsformen der Kollimatoreinheit sowie der diese umfassende Systeme sollen erfindungsgemäß nicht auf die Verwendung in dem beschriebenen Verfahren beschränkt werden. Auch Kollimatoreinheiten, die die beschriebenen Merkmale aufweisen, aber nicht für die Verwendung in dem Verfahren zum Beleuchten des Röntgenstrahlungsfeldes ausgebildet sind, seien von der Erfindung mit umfasst.

In einer weiteren Ausführungsform umfasst die Kollimatoreinheit wenigstens zwei Lamellen.

Die Lamellen können wie oben auch bezüglich des Verfahrens beschrieben ausgebildet sein.

Die wenigstens zwei Lamellen können so angeordnet sein, dass sie zusammen eine verstellbare Öffnung bilden, durch die die Röntgenstrahlung hindurchtreten kann. Durch Verstellen der Position und/oder des Abstands der Lamellen zueinander kann die Größe und Form dieser Öffnung angepasst werden, um das Röntgenstrahlungsfeld entsprechend zu begrenzen.

Die Lamellen können in verschiedenen Konfigurationen angeordnet sein. Beispielsweise können sie parallel zueinander angeordnet sein und sich in einer Ebene senkrecht zur Strahlungsrichtung bewegen. Alternativ können die Lamellen in einer V-förmigen oder anderen geometrischen Anordnung positioniert sein.

Die Lamellen können unabhängig voneinander oder synchron bewegt werden, um die Form und Größe des Röntgenstrahls präzise zu steuern. Insbesondere kann jede Lamelle individuell beweglich ausgebildet sein, um eine präzise Anpassung des Strahlungsfeldes zu ermöglichen. Die Bewegung der Lamellen kann durch verschiedene Mechanismen realisiert werden, wie etwa durch Linearantriebe, Schrittmotoren, piezoelektrische Aktuatoren, Elektromotoren, Piezoaktoren und/oder hydraulische Systeme.

In Ausführungen der Erfindung kann die Kollimatoreinheit mehr als zwei, insbesondere drei, vier, sechs, acht oder zehn Lamellen umfassen.

Die Verwendung von wenigstens zwei Lamellen ermöglicht eine flexible und präzise Anpassung des Röntgenstrahllungsfeldes an die spezifischen Anforderungen der jeweiligen Untersuchung. Dies kann dazu beitragen, die Strahlenexposition des Untersuchungsobjektes zu minimieren und gleichzeitig die Bildqualität zu optimieren. Vorteilhafterweise kann die Kollimatoreinheit mit wenigstens zwei Lamellen eine dynamische Anpassung des Röntgenstrahlungsfeldes während der Aufnahme ermöglichen. Insbesondere kann auf diese Weise das Röntgenstrahlungsfeld auch bei einer Bewegung der Röntgenstrahlungsquelle und/oder einer Bewegung des Fokus' der Röntgenstrahlung konstant, insbesondere ortsfest und/oder konstant in seiner Größe bzw. Ausdehnung und/oder Intensitätsverteilung, gehalten werden.

In einer weiteren Ausführungsform der Kollimatoreinheit sind die wenigstens zwei Lamellen unabhängig voneinander bewegbar.

Die unabhängige Bewegbarkeit der Lamellen kann auf verschiedene Weise realisiert werden. Beispielsweise kann jede Lamelle mit einem eigenen Antrieb versehen sein.

Alternativ oder zusätzlich können die Lamellen über separate Steuerungsmechanismen verfügen, die eine individuelle Positionierung jeder Lamelle ermöglichen. Diese Steuerungsmechanismen können beispielsweise mechanische Verbindungen, elektronische Steuersysteme oder eine Kombination aus beidem umfassen.

Optional können Endschalter oder Anschläge genutzt werden, die die maximale Bewegung der Lamellen begrenzen. Dies kann dazu beitragen, Kollisionen zwischen den Lamellen oder eine Überschreitung des zulässigen Bewegungsbereichs zu verhindern.

Die unabhängige Bewegbarkeit der beiden Lamellen ermöglicht es, auch bei verschiedenen Positionen der Röntgenquelle und/oder des Fokus' relativ zum Röntgendetektor und/oder dem Untersuchungsobjekt, das Röntgenstrahlungsfeld konstant zu halten. Insbesondere kann das Röntgenstrahlungsfeld für jede der Positionen ortsfest sein. Insbesondere kann eine Ausdehnung bzw. Größe des Röntgenstrahlungsfeldes für jede der Positionen konstant sein. Dies ist insbesondere bei der Tomosynthese von Bedeutung, da auf diese Weise die Bildqualität verbessert werden kann. Außerdem kann verhindert werden, dass durch die Bewegung der Röntgenquelle und/oder des Fokus' Bereiche des Untersuchungsobjektes bestrahlt werden, die nicht für die Bildgebung relevant sind.

Die unabhängige Bewegbarkeit kann es ermöglichen, asymmetrische Röntgenstrahlungsfelder zu erzeugen. Dies kann vorteilhaft sein, wenn bestimmte Bereiche des Untersuchungsobjektes einer höheren oder niedrigeren Strahlendosis ausgesetzt werden sollen. Zudem kann die unabhängige Bewegbarkeit eine dynamische Anpassung des Röntgenstrahlungsfeldes während der Aufnahme ermöglichen, beispielsweise um der Bewegung des Fokus oder der Röntgenquelle zu folgen. Die unabhängige Bewegbarkeit der Lamellen kann auch dazu genutzt werden, die Form des Röntgenstrahlungsfeldes dynamisch anzupassen. Beispielsweise können die Lamellen so positioniert werden, dass sie der Kontur des Untersuchungsobjektes folgen, was zu einer verbesserten Bildqualität und reduzierten Streustrahlung führen kann. Insbesondere kann durch die unabhängig voneinander bewegbaren Lamellen auf veränderte Geometrien beispielsweise durch die Bewegung der Röntgenquelle reagiert werden und das Röntgenstrahlungsfeld dennoch konstant gehalten werden. Vorteilhafterweise kann die unabhängige Bewegbarkeit der Lamellen eine präzisere Kontrolle über die Strahlendosis ermöglichen. Durch die Möglichkeit, die Größe und Form des Röntgenstrahlungsfeldes genau einzustellen, kann die Strahlenbelastung für das Untersuchungsobjekt minimiert werden, während gleichzeitig die Bildqualität optimiert wird. Zudem kann die unabhängige Bewegbarkeit der Lamellen die Flexibilität eines Mammographiesystems mit einer solchen Kollimatoreinheit erhöhen. Das Mammographiesystem kann leichter an verschiedene Untersuchungssituationen und Patientenanatomien angepasst werden, was zu einer verbesserten diagnostischen Genauigkeit führen kann.

In einer weiteren Ausführungsform der Kollimatoreinheit ist jede der Lamellen an einer Linearverstelleinheit angeordnet sein.

Insbesondere können wenigstens zwei Lamellen an verschiedenen Linearverstelleinheiten angeordnet sein. Alternativ oder zusätzlich können wenigstens zwei Lamellen an einer gemeinsamen Linearverstelleinheit angeordnet sein.

Die Linearverstelleinheiten können als eine mechanische Vorrichtung ausgebildet sein, die eine lineare Bewegung ermöglicht. Die Linearverstelleinheiten können dazu konfiguriert sein, eine präzise und kontrollierte Bewegung entlang einer geraden Linie zu ermöglichen. Die Linearverstelleinheiten können verschiedene Komponenten umfassen. Beispielsweise können die Linearverstelleinheiten eine Führungsschiene, einen Schlitten, einen Antrieb und ein Steuerungssystem umfassen. Die Führungsschiene kann als eine gerade Bahn ausgebildet sein, entlang derer sich der Schlitten bewegen kann. Der Schlitten kann als bewegliches Element ausgebildet sein, das die zu bewegende Last trägt. Der Antrieb kann als Motor, Hydraulikzylinder oder Pneumatikzylinder ausgebildet sein und kann die Kraft bereitstellen, um den Schlitten zu bewegen. Das Steuerungssystem kann dazu konfiguriert sein, die Bewegung des Schlittens präzise zu steuern. Die Linearverstelleinheiten können in verschiedenen Ausführungen realisiert sein. Beispielsweise können Linearführungen, Linearantriebe, lineare Positionier- und Handhabungssysteme oder Linearaktoren als Linearverstelleinheiten verwendet werden. Mögliche Ausführungsformen der Linearverstelleinheiten können Gewindespindeln, Zahnstangen, Riemenantriebe, Hydraulikzylinder oder Pneumatikzylinder umfassen. Die Wahl der spezifischen Art der Linearverstelleinheit kann von Faktoren wie der erforderlichen Präzision, Geschwindigkeit, Tragfähigkeit und dem verfügbaren Bauraum abhängen.

Die Linearverstelleinheiten können mit einem Steuersystem verbunden sein, welches die Bewegung der Lamellen koordiniert. Das Steuersystem kann die Position der Lamellen basierend auf Parametern wie der Bewegung der Röntgenquelle, der Fokusposition oder der gewünschten Kollimation des Röntgenstrahlungsfeldes berechnen und steuern.

Durch die Verwendung von Linearverstelleinheiten kann eine hohe Positioniergenauigkeit und Wiederholbarkeit der Lamellenbewegungen erreicht werden. Dies kann zu einer verbesserten Präzision der Kollimation und damit zu einer optimierten Strahlendosisverteilung führen. Vorteilhafterweise kann die Anordnung der Lamellen an Linearverstelleinheiten eine schnelle und dynamische Anpassung der Kollimation während der Röntgenaufnahme ermöglichen. Dies kann besonders vorteilhaft für Anwendungen sein, bei denen sich die Röntgenquelle relativ zum Röntgendetektor bewegt, wie beispielsweise bei der Tomosynthese. Die Anordnung von wenigstens zwei Lamellen an einer eigenen Linearverstelleinheit kann eine präzise und unabhängige Positionierung der einzelnen Lamellen ermöglichen. Dies kann eine flexible Anpassung der Kollimation an unterschiedliche Untersuchungssituationen und Röntgenstrahlungsfelder ermöglichen.

In einer weiteren Ausführungsform der Kollimatoreinheit ist wenigstens eine der Lamellen rotierbar ausgebildet.

Die rotierbare Lamelle kann um eine Achse drehbar gelagert sein. Die Rotationsachse kann beispielsweise senkrecht zur Hauptebene der Lamelle verlaufen. Insbesondere kann die Rotationsachse parallel zu einer Strahlachse der Röntgenstrahlung verlaufen. Die Strahlachse wird durch die Mittelachse des von der Röntgenquelle ausgesendeten Röntgenstrahlkegels definiert.

Die rotierbare Lamelle kann beispielsweise mittels eines Drehantriebs bewegt werden. Als Drehantrieb kann ein Elektromotor, ein Schrittmotor, ein Servomotor oder ein anderer geeigneter Antrieb verwendet werden. Die Rotationsbewegung kann kontinuierlich oder schrittweise erfolgen.

Die Rotationsbewegung der Lamelle kann mit der linearen Bewegung der Lamelle kombiniert werden.

Die Steuerung der Rotation kann in Abhängigkeit von der Bewegung der Röntgenquelle, der Bewegung des Fokus oder anderen Parametern der Röntgenuntersuchung erfolgen.

Insbesondere kann eine Lamelle, die am weitesten vom Untersuchungsobjekt entfernt ist, rotierbar ausgebildet sein. Mit anderen Worten kann die Lamelle, die einer Brustwand des Patienten gegenüber liegt, rotierbar ausgebildet sein. Mit anderen Worten kann die Lamelle rotierbar ausgebildet sein, die die Kante bzw. den Rand des Röntgenstrahlungsfeldes begrenzt, der der Brustwand gegenüberliegt.

Die Rotierbarkeit der Lamelle kann vorteilhaft sein, um das Röntgenstrahlungsfeld an unterschiedliche Untersuchungsobjekte oder Untersuchungsbereiche anzupassen. Durch Rotation kann die effektive Länge oder Breite der Lamelle variiert werden, was eine flexible Anpassung der Kollimation ermöglicht. Außerdem kann eine rotierbare Lamelle dazu beitragen, Kanten oder Ecken des Röntgenstrahlungsfeldes zu formen. Durch geeignete Rotation kann beispielsweise eine abgerundete oder abgeschrägte Kante des Röntgenstrahlungsfeldes erzeugt werden. Insbesondere kann eine durch die Bewegung der Röntgenquelle hervorgerufene Verzerrung des Röntgenstrahlungsfeldes an den Kanten durch die Rotation der Lamelle kompensiert werden. Außerdem kann durch die Rotation verhindert werden, dass durch die Bewegung der Röntgenquelle an der Lamelle vorbei Bereiche des Untersuchungsobjektes beleuchtet bzw. bestrahlt werden, die für die Bildgebung nicht relevant sind.

In einer weiteren Ausführungsform der Kollimatoreinheit weist wenigstens eine der Lamellen eine gekrümmte Kante auf.

Die gekrümmte Kante einer Lamelle kann als eine nicht-geradlinige Begrenzung der Lamelle ausgebildet sein. Die Krümmung kann konkav oder konvex sein. Die gekrümmte Kante kann beispielsweise als Kreisbogen, Ellipsenbogen, Parabelbogen oder eine andere gekrümmte Form ausgebildet sein.

Die Krümmung der Kante kann fest vorgegeben oder variabel ausgebildet sein. Bei einer variablen Krümmung kann die Form der Kante beispielsweise durch einen Aktuator oder einen Mechanismus angepasst werden, um verschiedene Untersuchungsobjekte oder Aufnahmesituationen zu berücksichtigen.

Die gekrümmte Kante kann dazu dienen, die Form des Röntgenstrahlungsfeldes an eine nichtrechteckige Form eines Untersuchungsobjekts anzupassen. Beispielsweise kann eine gekrümmte Kante verwendet werden, um das Röntgenstrahlungsfeld an die Kontur einer Brust bei einer Mammographie-Untersuchung anzupassen. Dies kann ermöglichen, dass das Röntgenstrahlungsfeld optimal auf den relevanten Bereich der Brust fokussiert wird, während umliegendes Gewebe weniger Strahlung ausgesetzt wird. Die gekrümmte Kante kann auch dazu beitragen, Streustrahlungseffekte zu reduzieren, indem sie eine bessere Anpassung an die Form des Untersuchungsobjekts ermöglicht. Dies kann zu einer verbesserten Bildqualität und einer reduzierten Strahlenbelastung für das Untersuchungsobjekt führen. Ferner kann eine gekrümmte Kante dazu beitragen, eine gleichmäßigere Beleuchtung des Röntgenstrahlungsfeldes zu erreichen, indem sie eine graduelle Änderung der Strahlungsintensität am Rand des Feldes ermöglicht.

In einer weiteren Ausführungsform der Kollimatoreinheit weist wenigstens eine der Lamellen eine variable Dicke auf.

Die variable Dicke einer Lamelle kann sich entlang der Länge, Breite und/oder Höhe der Lamelle ändern. Eine Lamelle mit variabler Dicke kann beispielsweise an einem Ende dicker sein als am anderen Ende oder in der Mitte dicker sein als an den Rändern. Beispielsweise kann die Dicke der Lamelle an einem Ende größer sein als am anderen Ende, wodurch ein keilförmiges Profil entsteht. Alternativ kann die Dicke der Lamelle in der Mitte größer oder kleiner sein als an den Rändern, was zu einem konvexen oder konkaven Profil führt.

Die variable Dicke einer Lamelle kann durch verschiedene Fertigungsverfahren erreicht werden. Beispielsweise kann die Lamelle gegossen, gefräst oder 3D-gedruckt werden, um die gewünschte Dickenvariation zu erzielen. Alternativ kann eine Lamelle mit variabler Dicke auch durch das Zusammenfügen mehrerer Schichten oder Segmente mit unterschiedlicher Dicke hergestellt werden.

Die Lamelle mit variabler Dicke kann verschiedene Funktionen erfüllen. Sie kann beispielsweise dazu dienen, den Röntgenstrahl gezielt zu formen oder zu filtern. Durch die Variation der Dicke kann die Intensität des Röntgenstrahls an verschiedenen Stellen des Röntgenstrahlungsfeldes angepasst werden. Die Lamelle mit variabler Dicke kann dazu beitragen, Streueffekte zu reduzieren oder den Kontrast in bestimmten Bereichen des Röntgenbildes zu verbessern. Die genaue Ausgestaltung der Dickenvariation kann dabei auf die spezifischen Anforderungen der jeweiligen Anwendung abgestimmt werden. Vorteilhafterweise kann durch die Verwendung von Lamellen mit variabler Dicke die Präzision und Flexibilität der Röntgenstrahlkollimation verbessert werden. Dies kann zu einer optimierten Bildgebung bei gleichzeitiger Minimierung der Strahlenbelastung führen, was sowohl die diagnostische Qualität als auch die Patientensicherheit erhöhen kann.

In einer weiteren Ausführungsform der Kollimatoreinheit ist die Dicke der wenigstens einen Lamelle derart ausgebildet, dass eine räumliche Inhomogenität der ausgesendeten Röntgenstrahlung am Röntgendetektor kompensiert werden kann.

Eine räumliche Inhomogenität der ausgesendeten Röntgenstrahlung kann eine ungleichmäßige Verteilung der Intensität der Röntgenstrahlung über das Röntgenstrahlungsfeld bezeichnen. Diese Inhomogenität kann durch verschiedene Faktoren verursacht werden, wie beispielsweise den Heel-Effekt oder Streuung der Röntgenstrahlung.

Die Kompensation der räumlichen Inhomogenität kann dadurch erreicht werden, dass Bereiche der Lamelle, die einer höheren Intensität der Röntgenstrahlung bzw. Strahlungsintensität ausgesetzt sind, eine größere Dicke aufweisen, während Bereiche der Lamelle für eine geringere Strahlungsintensität eine geringere Dicke aufweisen können. Dadurch kann eine gleichmäßigere Verteilung der Strahlungsintensität am Röntgendetektor erzielt werden.

Die Ausbildung der Dicke der Lamelle kann verschiedene Formen annehmen. Beispielsweise kann die Lamelle einen keilförmigen Querschnitt aufweisen, wobei die Dicke kontinuierlich von einem Ende zum anderen zunimmt. Alternativ kann die Dicke stufenweise variieren oder eine komplexere Kontur aufweisen, die speziell auf das Strahlungsprofil der Röntgenquelle abgestimmt ist.

Insbesondere kann die Lamelle einzelne Schichten umfassen, die relativ zueinander beweglich sind. Durch ein geeignetes Stapeln der Schichten kann die Dicke der Lamelle variiert und angepasst werden.

Die Kompensation der räumlichen Inhomogenität durch die variable Dicke der Lamelle kann in Kombination mit anderen Methoden zur Strahlungsformung eingesetzt werden, wie beispielsweise der Verwendung von Filtern oder der Anpassung der Röntgenquellenparameter.

Ein Steuersystem der Röntgenvorrichtung kann dazu konfiguriert sein, die Position und Ausrichtung der Lamelle mit variabler Dicke basierend auf Parametern wie der Röhrenspannung, dem Röhrenstrom oder der Geometrie der Röntgenquelle anzupassen, um eine optimale Kompensation der Strahlungsinhomogenität bzw. Intensitätsinhomogenitäten zu erreichen.

Vorteilhafterweise kann durch die Kompensation der räumlichen Inhomogenität der Röntgenstrahlung bzw. deren Intensität eine gleichmäßigere Belichtung des Röntgendetektors erreicht werden. Dies kann zu einer verbesserten Bildqualität führen, da Artefakte durch ungleichmäßige Belichtung reduziert werden können. Zudem kann eine gleichmäßigere Dosisverteilung im Untersuchungsobjekt erzielt werden, was aus Strahlenschutzgründen vorteilhaft sein kann.

Die Erfindung betrifft in einem dritten Aspekt ein Steuersystem. Das Steuersystem umfasst eine Recheneinheit und eine Schnittstelle. Die Recheneinheit und die Schnittstelle sind dazu ausgebildet, alle Schritte des computerimplementierten Verfahrens zum Beleuchten eines Röntgenstrahlungsfeldes auszuführen. Das computerimplementierte Verfahren kann die Schritte umfassen, wie sie in den vorhergehenden Ausführungsformen des computerimplementierten Verfahrens beschrieben wurden.

Das Steuersystem umfasst eine Recheneinheit und eine Schnittstelle. Die Recheneinheit kann als eine elektronische Verarbeitungseinheit ausgebildet sein, die dazu konfiguriert ist, Berechnungen durchzuführen und Steuerbefehle zu generieren. Die Schnittstelle kann als eine Kommunikationsschnittstelle ausgebildet sein, die dazu konfiguriert ist, Daten und Steuersignale zwischen der Recheneinheit und anderen Komponenten des Systems zu übertragen.

Die Recheneinheit und die Schnittstelle können dazu ausgebildet sein, alle Schritte des oben beschriebenen Verfahrens zum Beleuchten eines Röntgenstrahlungsfeldes auszuführen. Dies bedeutet, dass das Steuersystem in der Lage ist, die Bewegungen der Röntgenquelle, des Fokus' und der Kollimatoreinheit zu steuern und zu koordinieren, um eine konstante Beleuchtung des Röntgenstrahlungsfeldes während der Belichtungszeiträume zu gewährleisten. Dies kann umfassen, dass die Recheneinheit Steuerbefehle über die Schnittstelle an die verschiedenen Komponenten eines Röntgensystems sendet, um deren Bewegungen und Funktionen zu koordinieren.

Die Recheneinheit kann beispielsweise einen Mikroprozessor, einen Mikrocontroller, einen digitalen Signalprozessor (DSP) oder eine ähnliche Verarbeitungseinheit umfassen. Die Recheneinheit kann zudem einen Arbeitsspeicher und einen nichtflüchtigen Speicher umfassen, in dem Anweisungen und Daten zur Ausführung des Verfahrens gespeichert sein können.

Die Recheneinheit kann insbesondere eine Mehrzahl von Sub-Recheneinheiten umfassen, die jeweils zum Durchführen einzelner Verfahrensschritte ausgebildet sind.

Die Schnittstelle kann verschiedene Arten von Kommunikationsschnittstellen umfassen, wie zum Beispiel eine serielle Schnittstelle, eine parallele Schnittstelle, eine Netzwerkschnittstelle oder eine drahtlose Schnittstelle. Die Schnittstelle kann dazu ausgebildet sein, Steuersignale an die Röntgenquelle, die Fokussteuerung und die Kollimatoreinheit zu senden sowie Rückmeldungen und Messdaten von diesen Komponenten zu empfangen.

Die Schnittstelle kann eine Mehrzahl von Sub-Schnittstellen umfassen, die jeweils zum Durchführen einzelner Verfahrensschritte ausgebildet sind.

Das Steuersystem kann zudem eine Benutzeroberfläche umfassen, die es einem Bediener ermöglicht, Parameter des Verfahrens einzustellen und den Ablauf zu überwachen. Die Benutzeroberfläche kann beispielsweise einen Touchscreen, eine Tastatur, eine Maus oder andere Eingabegeräte umfassen.

Ferner kann das Steuersystem Softwaremodule umfassen, die spezifische Funktionen des Verfahrens implementieren, wie zum Beispiel Module zur Berechnung der Bewegungstrajektorien, zur Synchronisation der verschiedenen Komponenten und zur Bildverarbeitung.

Das Steuersystem kann als eigenständige Einheit ausgebildet sein oder in ein größeres System, wie beispielsweise ein Mammographiesystem, integriert sein. In letzterem Fall kann das Steuersystem mit anderen Subsystemen des Mammographiesystems kommunizieren und interagieren, um einen reibungslosen Gesamtablauf zu gewährleisten.

Die Recheneinheit kann beispielsweise dazu ausgebildet sein, Steuersignale zu generieren, um die Bewegung der Röntgenquelle relativ zum Röntgendetektor während der Belichtungszeiträume zu steuern. Weiterhin kann die Recheneinheit die Bewegung des Fokus' der Röntgenstrahlung relativ zur Bewegung der Röntgenquelle berechnen und entsprechende Steuersignale erzeugen.

Die Schnittstelle kann dazu ausgebildet sein, diese Steuersignale an die entsprechenden Komponenten wie Antriebe für die Röntgenquelle und den Fokus zu übermitteln. Zudem kann die Schnittstelle Sensordaten von den verschiedenen Komponenten empfangen, die von der Recheneinheit zur Steuerung und Überwachung des Verfahrens verwendet werden können.

Die Recheneinheit kann weiterhin dazu ausgebildet sein, die Bewegung der Kollimatoreinheit zu berechnen und zu steuern, um eine konstante Beleuchtung des Röntgenstrahlungsfeldes während der Belichtungszeiträume sicherzustellen. Dazu kann die Recheneinheit die Positionen und Bewegungen der Röntgenquelle, des Fokus' und der Kollimatoreinheit kontinuierlich erfassen und aufeinander abstimmen.

Die Schnittstelle kann zudem dazu ausgebildet sein, Benutzereingaben entgegenzunehmen, beispielsweise zur Konfiguration der Aufnahmeparameter oder zur Auswahl bestimmter Aufnahmemodi. Die Recheneinheit kann diese Eingaben verarbeiten und das Verfahren entsprechend anpassen.

Vorteilhafterweise ermöglicht das Steuersystem eine präzise und automatisierte Durchführung des Verfahrens zum Beleuchten eines Röntgenstrahlungsfeldes. Durch die koordinierte Steuerung aller beteiligten Komponenten kann eine optimale Bildqualität bei gleichzeitig minimierter Strahlenbelastung erreicht werden.

Die Erfindung betrifft in einem vierten Aspekt ein Mammographiesystem. Das Mammographiesystem umfasst eine Röntgenquelle, eine Kollimatoreinheit, eine Kompressionseinheit, einen Röntgendetektor und ein Steuersystem. Die Kollimatoreinheit kann wie oben beschrieben ausgebildet sein. Das Steuersystem kann wie oben beschrieben ausgebildet sein.

Die Röntgenquelle und der Röntgendetektor können wie bereits oben beschrieben ausgebildet sein.

Insbesondere kann die Röntgenquelle verschiedene Komponenten umfassen, die für die Erzeugung und Formung des Röntgenstrahls wichtig sind. Ein Emitter, oft eine Glühkathode, erzeugt einen Elektronenstrahl durch thermische Emission. Eine Fokussiereinheit kann den Elektronenstrahl bündeln und auf einen bestimmten Punkt der Anode lenken. Die Anode, typischerweise aus Wolfram oder Molybdän, dient als Ziel für den Elektronenstrahl und erzeugt bei dessen Auftreffen Röntgenstrahlung. Ein Röntgenfenster aus einem strahlungsdurchlässigen Material wie Beryllium ermöglicht den Austritt der erzeugten Röntgenstrahlung. Zusätzlich kann ein E-Catcher vorhanden sein, der unerwünschte Elektronen abfängt und so die Effizienz und Lebensdauer der Röntgenröhre verbessert. Diese Komponenten arbeiten zusammen, um einen präzisen und kontrollierbaren Röntgenstrahl für diagnostische oder therapeutische Zwecke zu erzeugen.

Die Kompressionseinheit kann als eine Vorrichtung ausgebildet sein, die das Untersuchungsobjekt, insbesondere eine menschliche Brust, komprimiert. Die Kompressionseinheit kann insbesondere wenigstens eine Kompressionsplatte umfassen. Insbesondere kann die Brust zwischen der Kompressionsplatte und einer Oberfläche des Röntgendetektors komprimiert werden. Insbesondere ist die Kompressionsplatte dafür verstellbar ausgebildet. Alternativ kann die Kompressionseinheit zwei Kompressionsplatten umfassen, zwischen denen die Brust komprimiert werden kann. Insbesondere kann dafür wenigstens eine Kompressionsplatte verstellbar ausgebildet sein. Die Kompressionsplatte bzw. die Kompressionsplatten kann aus einem röntgentransparenten Material wie Polycarbonat, Kohlenstofffaser oder PMMA gefertigt sein.

Die Komponenten des Mammographiesystems können so konfiguriert sein, dass sie zusammenwirken, um Bilddaten der Brust zu erzeugen. Beispielsweise kann die Röntgenquelle Röntgenstrahlung aussenden, die durch die komprimierte Brust transmittiert und vom Röntgendetektor erfasst wird, während die Kollimatoreinheit die Röntgenstrahlung formt und das Steuersystem den Prozess koordiniert. Der Röntgendetektor stellt die erfasste Röntgenstrahlung in Form von Bilddaten bereit.

Vorteilhafterweise kann das beschriebene Mammographiesystem eine verbesserte Bildqualität bei gleichzeitig reduzierter Strahlenbelastung für das Untersuchungsobjekt, insbesondere den Patienten, ermöglichen, auch bei einer Tomosynthese. Durch die präzise Steuerung der Kollimatoreinheit und die Abstimmung mit den anderen Komponenten kann das Röntgenstrahlungsfeld optimal an die Größe und Form der zu untersuchenden Brust und/oder an den Röntgendetektor angepasst werden. Dies kann zu einer Verringerung der Streustrahlung und einer Erhöhung des Bildkontrasts führen. Gleichzeitig kann die Strahlenexposition für das Untersuchungsobjekt minimiert werden, da nur der relevante Bereich bestrahlt wird.

Optional kann die Röntgenquelle außerdem einen E-Catcher umfassen. Ein E-Catcher, auch als Elektronenfänger bekannt, kann in Röntgenquellen verwendet werden, um unerwünschte Elektronen abzufangen. Diese Komponente kann dazu beitragen, die Effizienz und Lebensdauer der Röntgenröhre zu verbessern. Der E-Catcher kann so positioniert sein, dass er Elektronen auffängt, die nicht zur Erzeugung von Röntgenstrahlung beitragen, wie etwa rückgestreute oder fehlgeleitete Elektronen. In einigen Fällen kann der E-Catcher aus einem Material mit hoher Wärmeleitfähigkeit gefertigt sein, um die durch auftreffende Elektronen erzeugte Wärme effektiv abzuleiten. Die Verwendung eines E-Catchers kann dazu beitragen, unerwünschte Streustrahlung zu reduzieren und die Bildqualität in der Röntgendiagnostik zu verbessern.

Optional können in einem Röntgensystem und insbesondere in dem Mammographiesystem Filter verwendet werden, um das Energiespektrum des Röntgenstrahls zu modifizieren. Diese Filter können zwischen der Röntgenquelle und dem Untersuchungsobjekt positioniert werden, um bestimmte Energien des Röntgenspektrums selektiv abzuschwächen. Beispielsweise können Silber-, Kupfer-, Aluminium-, Titan- oder Rhodiumfilter oder Mischfilter eingesetzt werden, um niederenergetische Röntgenstrahlen zu entfernen, die sonst vom Gewebe des Untersuchungsobjektes absorbiert würden, ohne zur Bildgebung beizutragen. Diese Filterung kann dazu beitragen, den Bildkontrast zu optimieren und die Strahlendosis für das Untersuchungsobjekt zu reduzieren. In einigen Fällen kann die Filterung einstellbar sein, sodass das System verschiedene Filtermaterialien oder -dicken basierend auf den spezifischen Bildgebungsanforderungen oder Patienteneigenschaften auswählen kann. Die Verwendung einer geeigneten Filterung kann zu einer verbesserten Bildqualität beitragen und gleichzeitig dem Prinzip folgen, die Strahlenexposition so niedrig wie vernünftigerweise erreichbar zu halten (ALARA - As Low As Reasonably Achievable).

In optionalen Ausführungsformen kann ein Anti-Heel-Filter in der Röntgenquelle verwendet werden, um den sogenannten Heel-Effekt zu kompensieren. Der Heel-Effekt kann auftreten, wenn Röntgenstrahlen in einem Winkel zur Anodenoberfläche erzeugt werden, was zu einer ungleichmäßigen Intensitätsverteilung im Röntgenstrahl führen kann. Die Intensität kann auf der Kathodenseite des Strahlenfeldes höher sein als auf der Anodenseite. Ein Anti-Heel-Filter kann so gestaltet sein, dass er die Strahlung auf der Kathodenseite stärker abschwächt als auf der Anodenseite, um eine gleichmäßigere Intensitätsverteilung über das gesamte Bildfeld zu erreichen. Dies kann zu einer verbesserten Bildqualität und einer gleichmäßigeren Dosisverteilung im untersuchten Gewebe beitragen. Die genaue Ausgestaltung des Anti-Heel-Filters kann von Faktoren wie der Anodenwinkelung und den spezifischen Anforderungen des Bildgebungssystems abhängen.

In Röntgensystemen insbesondere in dem Mammographiesystem kann neben der Röntgenstrahlung aus dem Fokus auch extrafokale Röntgenstrahlung auftreten. Diese extrafokale Röntgenstrahlung kann durch Elektronen verursacht werden, die außerhalb des eigentlichen Fokusbereichs auf die Anode treffen oder durch Streuprozesse innerhalb der Röntgenquelle entstehen. Um die Auswirkungen dieser unerwünschten Röntgenstrahlung zu minimieren, können verschiedene Maßnahmen ergriffen werden. Beispielsweise können zusätzliche Kollimatoren oder Blenden eingesetzt werden, um die extrafokale Röntgenstrahlung zu blockieren. In einigen Fällen können auch spezielle Filterungen oder Abschirmungen verwendet werden, um die Intensität der extrafokalen Röntgenstrahlung zu reduzieren. Die Berücksichtigung und Kontrolle der extrafokalen Röntgenstrahlung kann dazu beitragen, die Bildqualität zu verbessern und die Strahlenbelastung für das Untersuchungsobjekt zu minimieren. In modernen Röntgensystemen können auch fortschrittliche Bildverarbeitungsalgorithmen eingesetzt werden, um die Auswirkungen der extrafokalen Röntgenstrahlung auf das resultierende Bild zu korrigieren.

Eine optionale Kontrolle der Strahlendosis in Röntgensystemen, insbesondere in dem Mammographiesystem, kann durch verschiedene Methoden erreicht werden. Ein bekannter Ansatz ist die Verwendung von automatischen Belichtungssteuerungen (AEC - Automatic Exposure Control). AEC-Systeme können die Röntgenstrahlungsintensität und -dauer basierend auf der Dichte und Dicke des Untersuchungsobjektes automatisch anpassen. Sie können Sensoren verwenden, um die Strahlungsmenge zu messen, die den Röntgendetektor erreicht, und die Belichtungsparameter entsprechend zu regulieren. Insbesondere kann eine Strahlendosis durch das Untersuchungsobjekt in einem Vorschuss (engl. Pre-Shot), welcher vor der eigentlichen Bildgebung mit einer typischerweise standardisierten, niedrigen Dosis erfasst wird, abgeschätzt werden und daraus die Belichtungsparameter abgeleitet werden. Dies kann dazu beitragen, eine optimale Bildqualität bei minimaler Strahlenbelastung für das Untersuchungsobjekt zu gewährleisten. Zusätzlich können moderne Röntgensysteme Vorfilterung und digitale Bildverarbeitungstechniken einsetzen, um die Strahlendosis weiter zu reduzieren. Die Kombination dieser Technologien mit sorgfältig optimierten Aufnahmeprotokollen kann eine effektive Dosiskontrolle ermöglichen und gleichzeitig die diagnostische Qualität der Röntgenaufnahmen aufrechterhalten.

In optionalen Ausführungsformen kann eine Dosiskontrolle in der Kollimatoreinheit, insbesondere in von dieser umfasste Lamellen, vorgesehen sein. Dabei können Sensoren direkt in den Lamellen oder in deren unmittelbarer Nähe angebracht sein, um die Strahlungsintensität präzise zu messen. Diese Integration ermöglicht eine genaue Kontrolle der von der Röntgenquelle ausgesendeten Strahlendosis. Die Kombination von Kollimation und Dosiskontrolle in einem Bauteil kann zudem die Komplexität des Gesamtsystems reduzieren und die Effizienz des Röntgensystems, insbesondere des Mammographiesystems, steigern.

Von der Kollimatoreinheit umfasste Lamellen können optional durch ein automatisches Kalibrierungsverfahren präzise eingestellt werden, indem ihre Position in Bilddaten gemessen wird. Das Röntgensystem, insbesondere das Mammographiesystem, kann dann Bilddaten aufnehmen, in dem der Röntgendetektor die Röntgenstrahlung während eines Belichtungszeitraums erfasst, und die Positionen der Lamellenkanten in den Bilddaten mittels Bildverarbeitungsalgorithmen automatisch detektieren. Dieser Prozess kann in regelmäßigen Intervallen oder bei Bedarf durchgeführt werden, um eine konstant präzise Kollimation zu gewährleisten. Die automatische Kalibrierung kann dazu beitragen, die Genauigkeit der Strahlenfeldeingrenzung auf das Röntgenstrahlungsfeld zu verbessern und somit die Bildqualität zu optimieren sowie die Strahlenexposition des Untersuchungsobjektes zu minimieren.

In einer Ausführungsform des Mammographiesystems ist die Röntgenquelle relativ zu dem Röntgendetektor beweglich ausgebildet.

Insbesondere kann die Röntgenquelle auch relativ zum Untersuchungsobjekt beweglich ausgebildet sein.

Die Bewegung der Röntgenquelle relativ zum Röntgendetektor kann auf verschiedene Arten realisiert werden. Die Röntgenquelle kann beispielsweise an einem beweglichen Arm oder einer Schwenkvorrichtung befestigt sein, die es ermöglicht, die Position der Röntgenquelle in Bezug auf den Röntgendetektor zu verändern. Alternativ kann der Röntgendetektor beweglich ausgebildet sein, während die Röntgenquelle an einer festen Position verbleibt. Eine weitere Möglichkeit besteht darin, sowohl die Röntgenquelle als auch den Röntgendetektor beweglich auszugestalten.

Die Bewegung der Röntgenquelle relativ zum Röntgendetektor kann in verschiedenen Richtungen erfolgen. So kann eine lineare Bewegung entlang einer oder mehrerer Achsen vorgesehen sein. Ebenso kann eine Rotationsbewegung bzw. eine Schwenkbewegung bzw. eine bogenförmige Bewegung um eine oder mehrere Achsen ermöglicht werden. Auch eine Kombination aus linearen und rotatorischen Bewegungen ist denkbar.

Die Bewegung der Röntgenquelle relativ zum Röntgendetektor kann durch verschiedene Antriebsmechanismen realisiert werden. Hierzu können beispielsweise elektrische Motoren, hydraulische oder pneumatische Systeme oder auch manuelle Verstellmechanismen zum Einsatz kommen.

Die Steuerung der relativen Bewegung kann mittels des Steuersystems erfolgen. Diese kann so konfiguriert sein, dass sie die Bewegung basierend auf vordefinierten Aufnahmeprotokollen oder benutzerdefinierten Einstellungen steuert.

Die Bewegung der Röntgenquelle relativ zum Röntgendetektor kann kontinuierlich oder schrittweise erfolgen. Bei einer kontinuierlichen Bewegung kann die Röntgenquelle während eines oder mehrerer Belichtungszeiträume entlang einer vordefinierten Bahn bewegt werden. Bei einer schrittweisen Bewegung können Bilddaten mittels des Röntgendetektors an diskreten Positionen erfasst werden. Dafür kann die Röntgenquelle zwischen einzelnen Belichtungszeiträumen bewegt werden und während eines Belichtungszeitraums fest stehen.

Die Bewegung der Röntgenquelle relativ zum Röntgendetektor und insbesondere relativ zum Untersuchungsobjekt kann vorteilhaft für verschiedene Aufnahmetechniken genutzt werden. So können beispielsweise Tomosynthese-Aufnahmen ermöglicht werden, bei denen die Röntgenquelle während der Aufnahme einen Bogen um das Untersuchungsobjekt beschreibt. Eine Aufnahme umfasst dabei insbesondere mehrere Belichtungszeiträume. Auch können durch die Beweglichkeit verschiedene Projektionswinkel realisiert werden, um bestimmte Strukturen im Untersuchungsobjekt besser darzustellen. Zudem kann die Bewegung dazu genutzt werden, die Röntgenquelle und den Röntgendetektor optimal an die Anatomie des Untersuchungsobjektes anzupassen. Dies kann zu einer verbesserten Bildqualität und einer reduzierten Strahlenbelastung für das Untersuchungsobjekt führen.

Vorteilhafterweise kann die Bewegung der Röntgenquelle relativ zum Röntgendetektor die diagnostische Aussagekraft der Aufnahmen erhöhen, indem sie die Aufnahme von Bildern aus verschiedenen Perspektiven ermöglicht. Dies kann insbesondere bei der Erkennung und Charakterisierung von Läsionen oder anderen Auffälligkeiten im Brustgewebe hilfreich sein. Insbesondere kann durch die Anwendung in der Tomosynthese eine dreidimensionale Darstellung bzw. Abbildung bzw. Röntgenbild des Untersuchungsobjektes erfasst bzw. bestimmt werden. Dieses kann insbesondere für eine Biopsie und insbesondere für eine Pfadplanung in der Biopsie genutzt werden.

In einer weiteren Ausführungsform des Mammographiesystems kann die Röntgenquelle um mehr als 30° relativ zu dem Röntgendetektor beweglich ausgebildet sein.

Der Winkelbereich von mehr als 30° kann beispielsweise 40°, 50°, 60° oder sogar bis zu 180° betragen.

Die Möglichkeit einer Bewegung der Röntgenquelle relativ zum Röntgendetektor um mehr als 30° kann durch entsprechende Steuer- und Regelungsmechanismen unterstützt werden, um präzise und reproduzierbare Bewegungen zu gewährleisten. Dies kann Positionssensoren, Motoren und eine spezialisierte Software umfassen.

Zusätzlich zur Beweglichkeit der Röntgenquelle kann optional auch der Röntgendetektor beweglich ausgebildet sein, um die relative Bewegung zwischen Röntgenquelle und Röntgendetektor zu optimieren. Dies kann eine noch flexiblere Positionierung und Bildgebung ermöglichen.

Alternativ kann ein Mammographiesystem mit mehreren Röntgenquellen verwendet werden, die in einem Bogen um das Untersuchungsobjekt angeordnet sind. In diesem Fall kann die Bewegung der Röntgenquelle durch sequenzielles Aktivieren der verschiedenen Röntgenquellen simuliert werden.

Die Möglichkeit einer Bewegung der Röntgenquelle um mehr als 30° relativ zu dem Röntgendetektor kann eine erweiterte Tomosynthese-Bildgebung ermöglichen. Bei der Tomosynthese-Bildgebung kann die Röntgenquelle in einem Bogen um das Untersuchungsobjekt bewegt werden, während mehrere Projektionsaufnahmen bzw. Bilddaten aus unterschiedlichen Winkeln aufgenommen werden. Ein größerer Winkelbereich kann dabei zu einer verbesserten Tiefenauflösung und Bildqualität von rekonstruierten dreidimensionalen Bilddaten führen. Die Möglichkeit einer Bewegung der Röntgenquelle um mehr als 30° relativ zum Röntgendetektor kann auch für andere Bildgebungsverfahren neben der Tomosynthese vorteilhaft sein. Beispielsweise kann sie für stereotaktische Biopsien oder für die Aufnahme von Bildern aus unkonventionellen Winkeln genutzt werden, um schwer zugängliche Bereiche besser darzustellen.

In einer weiteren Ausführungsform des Mammographiesystems kann die Röntgenquelle dazu ausgebildet sein, einen Fokus der von der Röntgenquelle ausgesendeten Röntgenstrahlung zu bewegen.

Der Fokus ist dabei wie oben beschrieben definiert und ausgebildet. Der Fokus wird dabei innerhalb der Röntgenquelle, insbesondere auf der Anode bewegt.

Die Bewegung des Fokus' kann dabei insbesondere kontinuierlich sein. Alternativ kann die Bewegung des Fokus' Stufen und/oder Sprünge umfassen.

Die Röntgenquelle kann eine Ablenkeinheit umfassen, um die Fokusbewegung zu ermöglichen. Ein Beispiel kann eine elektrostatische Ablenkeinheit sein, die den Elektronenstrahl innerhalb der Röntgenquelle ablenkt bzw. steuert. Alternativ oder zusätzlich kann eine magnetische Ablenkeinheit den Elektronenstrahl in der Röntgenquelle steuern bzw. ablenken. Mögliche Ausführungsformen der Ablenkeinheit umfassen Elektromagnete, elektrostatische Ablenkplatten oder Spulen. Die entsprechende Ablenkeinheit kann dabei außerhalb oder innerhalb der Röntgenquelle angeordnet sein. Insbesondere kann die Ablenkeinheit in einem Eintank der Röntgenquelle oder in einem Vakuumgehäuse der Röntgenquelle angeordnet sein. Ein anderes Beispiel kann eine mechanische Ablenkeinheit sein, die die Position der Anode oder des gesamten Röhrenkopfes kontinuierlich verändert.

Die Fokusbewegung kann in verschiedenen Mustern erfolgen. Beispiele können eine lineare Bewegung, eine kreisförmige Bewegung, eine elliptische Bewegung, eine treppenförmige Bewegung, eine sägezahnförmige Bewegung oder eine komplexere Trajektorie umfassen. Die Wahl des Bewegungsmusters kann von den spezifischen Anforderungen der Bildgebung abhängen.

Die Geschwindigkeit der Fokusbewegung kann variabel sein und kann an die Bildaufnahmeparameter angepasst werden.

Die Fokusbewegung kann mit der Bewegung der Röntgenquelle relativ zum Röntgendetektor koordiniert werden. Dies kann ermöglichen, dass der Fokus aus Sicht des Röntgendetektors ortsfest erscheint, während sich die Röntgenquelle bewegt.

Die Steuerung der Fokusbewegung kann durch das Steuersystem des Mammographiesystems erfolgen. Das Steuersystem kann so konfiguriert sein, dass sie die Fokusbewegung basierend auf Parametern wie der Bewegungsgeschwindigkeit und -richtung der Röntgenquelle, der gewünschten Bildauflösung und/oder der Geometrie des Untersuchungsobjektes steuert.

Die Fähigkeit zur Fokusbewegung kann verschiedene Vorteile bieten. Sie kann beispielsweise die Bildqualität verbessern, indem sie die effektive Fokusgröße reduziert und damit die räumliche Auflösung erhöht. Alternativ kann der Fokus derart bewegt werden, dass er aus Sicht des Röntgendetektors eine gewünschte Form annimmt. Dies kann ebenfalls zur Optimierung der Bildqualität beitragen.

In einer weiteren Ausführungsform des Mammographiesystems ist die Kollimatoreinheit auf einer Achse zwischen Röntgenquelle und Röntgendetektor verfahrbar ausgebildet.

Die Achse zwischen Röntgenquelle und Röntgendetektor kann als eine gedachte Linie verstanden werden, die die Röntgenquelle mit dem Röntgendetektor verbindet. Diese Achse kann auch als Strahlachse bzw. Strahlungsachse oder optische Achse bezeichnet werden.

Verfahrbar ausgebildet bedeutet, dass die Kollimatoreinheit entlang dieser Achse bewegt werden kann. Die Kollimatoreinheit kann beispielsweise auf Schienen oder in Führungen angeordnet sein, die eine Bewegung entlang der Achse ermöglichen. Die Kollimatoreinheit kann beispielsweise mittels eines Linearantriebs oder eines Elektromotors mit Spindelantrieb entlang der Achse bewegt werden. Alternativ kann die Bewegung auch manuell durch einen Bediener erfolgen. Die Verfahrbarkeit kann kontinuierlich oder in diskreten Schritten ausgebildet sein.

Die Verfahrbarkeit der Kollimatoreinheit kann optional mit Sensoren gekoppelt sein, die die aktuelle Position der Kollimatoreinheit erfassen. Diese Positionsinformation kann von einem Steuersystem genutzt werden, um die Kollimation optimal einzustellen.

Die Position der Kollimatoreinheit auf der Achse kann abhängig von verschiedenen Faktoren gewählt werden. Zum Beispiel kann die Position an die Größe des Untersuchungsobjektes, eine gewünschte Vergrößerung oder eine Strahlgeometrie angepasst werden. Eine Positionierung näher an der Röntgenquelle kann eine schärfere Begrenzung des Röntgenstrahlungsfeldes ermöglichen, während eine Position näher am Röntgendetektor eine feinere Anpassung des Röntgenstrahlungsfeldes an die Röntgendetektorfläche erlauben kann.

Das Steuersystem kann dazu ausgebildet sein, die Position der Kollimatoreinheit in Abhängigkeit von verschiedenen Parametern wie der aktuellen Position der Röntgenquelle, der Fokusposition oder der gewünschten Röntgenstrahlungsfeldgröße anzupassen. Dies ermöglicht eine präzise und automatisierte Steuerung der Kollimation.

Vorteilhafterweise kann durch die Verfahrbarkeit der Kollimatoreinheit eine optimale Anpassung der Kollimation an verschiedene Aufnahmesituationen und Untersuchungsobjekte erreicht werden. Dies kann zu einer Verbesserung der Bildqualität und einer Reduzierung der Strahlendosis für den Patienten führen, da das Strahlungsfeld genau auf den relevanten Bereich begrenzt werden kann. Insbesondere bietet die Verfahrbarkeit entlang der Achse einen alternativen oder einen zusätzlichen Freiheitsgrad, um Form und/oder Größe des Röntgenstrahlungsfeldes zu variieren und anzupassen.

In einer weiteren Ausführungsform des Mammographiesystems kann die Kollimatoreinheit in einem Eintank der Röntgenquelle angeordnet sein.

Der Eintank kann als ein geschlossenes Gehäuse ausgebildet sein, das die Röntgenquelle umgibt und verschiedene Komponenten der Röntgenquelle beinhaltet. Der Eintank kann dazu konfiguriert sein, die Röntgenquelle von der Umgebung abzuschirmen und eine kontrollierte Umgebung für den Betrieb der Röntgenquelle bereitzustellen.

Der Eintank kann verschiedene Komponenten umfassen, wie beispielsweise eine Anode, eine Kathode, ein Röntgenfenster und Kühlvorrichtungen. Die Kollimatoreinheit kann innerhalb des Eintanks so positioniert sein, dass sie den Röntgenstrahl unmittelbar nach dessen Erzeugung kollimiert, bevor dieser den Eintank durch das Röntgenfenster verlässt.

Der Eintank kann mit einem isolierenden Kühlmittel, insbesondere einem Öl, gefüllt sein, welches zur Kühlung der Röntgenquelle dient. Durch die Anordnung der Kollimatoreinheit im Eintank kann diese ebenfalls von dem Kühlmittel umgeben sein. Dies kann eine effektive Kühlung der Kollimatoreinheit ermöglichen, was besonders bei hohen Strahlungsintensitäten vorteilhaft sein kann.

Die Anordnung der Kollimatoreinheit im Eintank kann verschiedene Vorteile bieten. Zum einen kann dadurch eine kompakte Bauweise der Röntgenquelle ermöglicht werden, da die Kollimatoreinheit direkt in die Röntgenquelle integriert ist. Zum anderen kann die Kollimatoreinheit durch die Anordnung im Eintank vor äußeren Einflüssen geschützt werden. Die Anordnung im Eintank kann es zudem ermöglichen, die Bewegung der Kollimatoreinheit präzise mit der Bewegung des Fokus der Röntgenquelle zu synchronisieren, da beide Komponenten in derselben abgeschlossenen Umgebung untergebracht sind. Dies kann zu einer verbesserten Genauigkeit und Effizienz der Kollimation beitragen. Die Integration der Kollimatoreinheit in den Eintank kann auch den Schutz vor Streustrahlung verbessern. Der Eintank kann aus strahlungsabsorbierendem Material gefertigt sein, wodurch die Streustrahlung, die von der Kollimatoreinheit erzeugt wird, effektiv abgeschirmt werden kann. Die Integration der Kollimatoreinheit in den Eintank kann auch die elektrische und mechanische Verbindung zur Röntgenquelle vereinfachen. Steuer- und Versorgungsleitungen können innerhalb des Eintanks geführt werden, was die Komplexität des Gesamtsystems reduzieren kann. Die verbesserte Kühlung, der erhöhte Strahlenschutz und die vereinfachte Wartung können die Leistungsfähigkeit und Zuverlässigkeit des Systems steigern.

In einer weiteren Ausführungsform der Kollimatoreinheit kann die Kollimatoreinheit vor einem Röntgenaustrittsfenster der Röntgenquelle angeordnet sein.

Das Röntgenaustrittsfenster kann als eine Öffnung in einem Gehäuse der Röntgenquelle ausgebildet sein, durch welche die von der Röntgenquelle erzeugte Röntgenstrahlung austreten kann. Die Anordnung der Kollimatoreinheit vor dem Röntgenaustrittsfenster kann ermöglichen, dass die Röntgenstrahlung unmittelbar nach Austritt aus der Röntgenquelle kollimiert wird.

Die Kollimatoreinheit kann in einem definierten Abstand vor dem Röntgenaustrittsfenster angeordnet sein. Dieser Abstand kann beispielsweise zwischen 1 mm und 100 mm betragen. Insbesondere kann die Kollimatoreinheit auch direkt vor dem Röntgenfenster angeordnet sein. Insbesondere kann die Kollimatoreinheit an die Röntgenquelle angeflanscht sein. Die genaue Positionierung der Kollimatoreinheit relativ zum Röntgenaustrittsfenster kann von verschiedenen Faktoren abhängen, wie etwa der Größe des Röntgenaustrittsfensters, der Divergenz des Röntgenstrahls oder der gewünschten Größe des kollimierten Röntgenstrahlungsfeldes.

Die Kollimatoreinheit kann fest vor dem Röntgenaustrittsfenster montiert sein oder kann relativ zum Röntgenaustrittsfenster beweglich und/oder lösbar ausgebildet sein.

Durch die Anordnung vor dem Röntgenaustrittsfenster kann die Kollimatoreinheit als ein separates Bauteil von der Röntgenquelle ausgebildet sein. Dies kann Vorteile bei der Wartung oder dem Austausch der Kollimatoreinheit bieten, da diese unabhängig von der Röntgenquelle zugänglich sein kann. Die Anordnung der Kollimatoreinheit vor dem Röntgenaustrittsfenster kann im Vergleich zu einer Anordnung innerhalb der Röntgenquelle den Vorteil bieten, dass die Kollimatoreinheit nicht den hohen Temperaturen und der intensiven Strahlung im Inneren der Röntgenquelle ausgesetzt ist. Dies kann die Lebensdauer der Kollimatoreinheit verlängern und die Verwendung von Materialien ermöglichen, die für eine Anwendung im Inneren der Röntgenquelle nicht geeignet wären. Die Positionierung vor dem Röntgenaustrittsfenster kann ermöglichen, dass die Kollimatoreinheit die Röntgenstrahlung unmittelbar nach deren Austritt aus der Röntgenquelle begrenzt. Dies kann zu einer präziseren Kontrolle des Röntgenstrahlungsfeldes führen, da die Röntgenstrahlung weniger Gelegenheit hat, sich vor der Kollimation auszubreiten. Weiterhin kann diese Konfiguration es ermöglichen, die Kollimatoreinheit flexibel an verschiedene Röntgenquellen anzupassen oder auszutauschen, ohne in die interne Struktur der Röntgenquelle eingreifen zu müssen. Dies kann die Modularität und Anpassungsfähigkeit des gesamten Röntgensystems erhöhen. Die Positionierung vor dem Röntgenaustrittsfenster kann somit zu einer verbesserten Gesamtleistung und Benutzerfreundlichkeit des Röntgensystems insbesondere des Mammographiesystems beitragen.

In einer weiteren Ausführungsform des Mammographiesystems umfasst die Kompressionseinheit wenigstens eine Kompressionsplatte. Dabei ist die Kollimatoreinheit auf der Kompressionsplatte oder auf dem Röntgendetektor angeordnet.

Die Kompressionsplatte kann dabei insbesondere wie oben beschrieben ausgebildet sein.

Der Röntgendetektor kann wie oben beschrieben ausgebildet sein.

Die Anordnung der Kollimatoreinheit auf der Kompressionsplatte kann so ausgebildet sein, dass die Kollimatoreinheit fest mit der Kompressionsplatte verbunden ist oder abnehmbar auf dieser angebracht werden kann. Im Fall einer abnehmbaren Anordnung kann die Kollimatoreinheit beispielsweise über Klemmvorrichtungen, Magnete oder andere geeignete Befestigungsmechanismen an der Kompressionsplatte befestigt werden.

Bei einer Anordnung der Kollimatoreinheit auf dem Röntgendetektor kann die Kollimatoreinheit direkt auf der der Röntgenquelle zugewandten Oberfläche des Röntgendetektors montiert sein oder in einem geringen Abstand darüber angebracht werden. Die Befestigung kann dabei permanent oder abnehmbar ausgeführt sein, um eine flexible Nutzung und einfache Wartung zu ermöglichen.

Die Anordnung der Kollimatoreinheit auf der Kompressionsplatte oder dem Röntgendetektor kann zudem eine kompakte Bauweise des Mammographiesystems ermöglichen. Es kann weniger Platz zwischen Röntgenquelle und Röntgendetektor benötigt werden, da die Kollimatoreinheit nicht als separate Komponente im Strahlengang angeordnet sein muss. Vorteilhafterweise kann diese Konfiguration eine flexible Anpassung der Kollimation an verschiedene Untersuchungssituationen ermöglichen. Die Kollimatoreinheit kann beispielsweise leicht ausgetauscht oder angepasst werden, wenn sie auf der Kompressionsplatte oder dem Röntgendetektor montiert ist. Zudem kann diese Anordnung eine verbesserte Wartung und Kalibrierung der Kollimatoreinheit ermöglichen, da sie leichter zugänglich sein kann als bei einer Positionierung näher an der Röntgenquelle. Ein weiterer technischer Vorteil dieser Ausführungsform kann darin bestehen, dass die Kollimation besser an die Form und Größe des komprimierten Gewebes des Untersuchungsobjektes angepasst werden kann, wenn die Kollimatoreinheit direkt auf der Kompressionsplatte oder dem Röntgendetektor angeordnet ist. Dies kann zu einer optimierten Strahlendosisverteilung und einer verbesserten Bildqualität führen. Zudem ist eine auf der Kompressionsplatte oder dem Röntgendetektor angeordnete Kollimationseinheit günstiger als eine Kollimationseinheit im Eintank oder am Strahlenaustrittsfenster der Röntgenquelle, da die Anforderungen and Präzision nicht so hoch sind.

In einer weiteren Ausführungsform des Mammographiesystems umfasst die Kollimatoreinheit wenigstens eine erste und eine zweite Sub-Einheit. Die erste Sub-Einheit ist in einem ersten Abstand zu dem Röntgendetektor angeordnet. Die zweite Sub-Einheit ist in einem zweiten Abstand zu dem Röntgendetektor angeordnet. Der erste und der zweite Abstand sind verschieden.

Der erste Abstand kann als ein räumlicher Abstand zwischen der ersten Sub-Einheit und dem Röntgendetektor ausgebildet sein. Der zweite Abstand kann als ein räumlicher Abstand zwischen der zweiten Sub-Einheit und dem Röntgendetektor ausgebildet sein. Verschieden kann bedeuten, dass die Abstände unterschiedliche Werte aufweisen.

Die erste und zweite Sub-Einheit können jeweils als separate Kollimatorkomponenten insbesondere als separate Kollimatoreinheiten, ausgebildet sein. Jede Sub-Einheit kann eine oder mehrere Lamellen umfassen. Insbesondere können die erste und die zweite Sub-Einheit jeweils wie oben bezüglich der Kollimatoreinheit beschrieben ausgebildet sein. Die erste und die zweite Sub-Einheit können gemäß gleicher oder verschiedener Ausführungsformen der Kollimatoreinheit ausgebildet sein. Mit anderen Worten können die beiden Sub-Einheiten in Form einer gleichen Ausführungsform der oben beschriebenen Kollimatoreinheit ausgebildet sein. Alternativ können sie als verschiedenen Ausführungsformen der Kollimatoreinheit ausgebildet sein. Die erste und die zweite Sub-Einheiten können in unterschiedlichen Ebenen zwischen der Röntgenquelle und dem Röntgendetektor angeordnet sein. Insbesondere kann jede der Sub-Einheiten an einer der oben beschriebenen Positionen (im Eintank, vor dem Röntgenaustrittsfenster und/oder auf der Kompressionsplatte oder dem Röntgendetektor) angeordnet sein.

Der erste Abstand kann größer als der zweite Abstand sein. In diesem Fall kann die erste Sub-Einheit näher an der Röntgenquelle angeordnet sein als die zweite Sub-Einheit. Alternativ kann der zweite Abstand größer als der erste Abstand sein. In diesem Fall kann die zweite Sub-Einheit näher an der Röntgenquelle angeordnet sein als die erste Sub-Einheit.

Der erste und der zweite Abstand der ersten bzw. der zweiten Sub-Einheit können so gewählt werden, dass eine optimale Kollimation für verschiedene Untersuchungsobjekte oder Bildgebungsmodi erreicht wird. Beispielsweise kann die erste Sub-Einheit nahe der Röntgenquelle angeordnet sein, während die zweite Sub-Einheit näher am Untersuchungsobjekt oder dem Röntgendetektor positioniert ist.

Bewegungen der Lamellen der ersten und/oder zweiten Sub-Einheit können aufeinander abgestimmt sein, um eine präzise Steuerung bzw. Kollimierung bzw. Begrenzung des Röntgenstrahlungsfeldes zu ermöglichen. Das Steuersystem kann dazu ausgebildet sein, die Bewegungen der Lamellen in beiden Sub-Einheiten koordinieren, um eine optimale Kollimation zu erreichen.

Die Anordnung von mehreren Sub-Einheiten in unterschiedlichen Abständen zum Röntgendetektor kann eine präzisere Kollimation des Röntgenstrahls ermöglichen. Die erste Sub-Einheit kann beispielsweise für eine grobe Kollimation ausgebildet sein, während die zweite Sub-Einheit für eine feinere Kollimation ausgebildet sein kann. Dies kann zu einer verbesserten Bildqualität und einer reduzierten Strahlenbelastung für den Patienten führen.

Die Erfindung betrifft in einem fünften Aspekt ein Computerprogrammprodukt mit einem Computerprogramm, welches direkt in einen Speicher eines Steuersystems ladbar ist, mit Programmabschnitten, um alle Schritte des oben beschriebenen computerimplementierten Verfahrens zum Beleuchten eines Röntgenstrahlungsfeldes auszuführen, wenn die Programmabschnitte von dem Steuersystem ausgeführt werden.

Ein Computerprogramm kann als eine Folge von Anweisungen ausgebildet sein, die von einem Computer ausgeführt werden können, um eine bestimmte Aufgabe oder Funktion zu erfüllen. Das Computerprogramm kann in einer oder mehreren Programmiersprachen geschrieben sein.

Die Programmabschnitte können in einer Programmiersprache wie C++, Java oder Python implementiert sein.

Ein Speicher eines Steuersystems kann als ein Datenspeicher ausgebildet sein, in den das Computerprogramm geladen und aus dem es ausgeführt werden kann. Der Speicher kann beispielsweise als Festplattenspeicher, Solid-State-Drive, RAM oder ROM ausgebildet sein.

Programmabschnitte können als Teile oder Module des Computerprogramms ausgebildet sein, die bestimmte Funktionen oder Aufgaben erfüllen. Die Programmabschnitte können beispielsweise als Funktionen, Klassen, Methoden oder Prozeduren implementiert sein.

Das Steuersystem kann als ein Computersystem ausgebildet sein, das dazu konfiguriert ist, das Computerprogramm auszuführen und die Schritte des Verfahrens zu steuern. Das Steuersystem kann beispielsweise einen Prozessor, Arbeitsspeicher und Schnittstellen umfassen.

Das Laden des Computerprogramms in den Speicher kann als ein Vorgang ausgebildet sein, bei dem das Programm von einem Speichermedium in den Arbeitsspeicher des Steuersystems übertragen wird, sodass es ausgeführt werden kann.

Die Ausführung der Programmabschnitte durch das Steuersystem kann als ein Prozess ausgebildet sein, bei dem der Prozessor des Steuersystems die Anweisungen des Computerprogramms abarbeitet und die entsprechenden Operationen durchführt.

Das Computerprogrammprodukt kann als Software auf einem Datenträger oder als Download über ein Netzwerk bereitgestellt werden.

Vorteilhafterweise kann durch die Bereitstellung als Computerprogrammprodukt eine flexible Anpassung und Aktualisierung der Steuerungssoftware ermöglicht werden. Neue Funktionen oder optimierte Algorithmen können einfach durch ein Software-Update eingespielt werden, ohne dass Hardware-Änderungen am Mammographiesystem erforderlich sind.

Eine weitgehend softwaremäßige Realisierung hat den Vorteil, dass auch schon bisher verwendete Steuersysteme auf einfache Weise durch ein Software-Update nachgerüstet werden können, um auf die beschriebene Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Die Erfindung betrifft in einem sechsten Aspekt ein computerlesbares Speichermedium, auf welchem von einem Steuersystem lesbare und ausführbare Programmabschnitte gespeichert sein können, um alle Schritte des oben beschriebenen computerimplementierten Verfahrens zum Beleuchten eines Röntgenstrahlungsfeldes auszuführen, wenn die Programmabschnitte von dem Steuersystem ausgeführt werden.

Ein computerlesbares Speichermedium kann als ein nicht-flüchtiges Speichermedium ausgebildet sein, das dazu konfiguriert ist, Daten oder Anweisungen dauerhaft zu speichern. Das computerlesbare Speichermedium kann beispielsweise als eine Festplatte, ein Solid-State-Laufwerk, ein USB-Stick, eine SD-Karte oder ein optisches Speichermedium wie eine CD oder DVD ausgebildet sein.

Von einem Steuersystem lesbare und ausführbare Programmabschnitte können als Softwarecode oder Computerprogrammcode ausgebildet sein, der von einem Prozessor oder einer Recheneinheit des Steuersystems interpretiert und ausgeführt werden kann. Die Programmabschnitte können in einer Programmiersprache wie C++, Java oder Python geschrieben sein.

Das Steuersystem kann als ein Computersystem, ein eingebettetes System oder ein spezialisiertes Steuergerät ausgebildet sein, das dazu konfiguriert ist, die auf dem computerlesbaren Speichermedium gespeicherten Programmabschnitte auszuführen. Das Steuersystem kann einen Prozessor, Arbeitsspeicher und Schnittstellen umfassen, um die Programmabschnitte zu laden und auszuführen.

Das Ausführen der Programmabschnitte durch das Steuersystem kann dazu führen, dass das Steuersystem alle Schritte des in den vorherigen Ansprüchen beschriebenen Verfahrens durchführt. Dies kann das Bewegen einer Röntgenquelle, das Bewegen eines Fokus der Röntgenstrahlung und das Bewegen einer Kollimatoreinheit umfassen, um ein Röntgenstrahlungsfeld konstant zu beleuchten.

Durch die Speicherung der Programmabschnitte auf einem computerlesbaren Speichermedium kann das Verfahren flexibel auf verschiedenen Steuersystemen implementiert werden. Das computerlesbare Speichermedium kann einfach in ein bestehendes System integriert oder ausgetauscht werden, um das Verfahren zu aktualisieren oder zu modifizieren.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen:
FIG. 1 ein erstes Ausführungsbeispiel eines Verfahrens zum Beleuchten eines Röntgenstrahlungsfeldes,
FIG. 2 ein zweites Ausführungsbeispiel eines Verfahrens zum Beleuchten eines Röntgenstrahlungsfeldes,
FIG. 3a ein erstes Ausführungsbeispiel einer Kollimatoreinheit,
FIG. 3b ein zweites Ausführungsbeispiel einer Kollimatoreinheit,
FIG. 3c ein drittes Ausführungsbeispiel einer Kollimatoreinheit,
FIG. 3d ein viertes Ausführungsbeispiel einer Kollimatoreinheit,
FIG. 3e ein fünftes Ausführungsbeispiel einer Kollimatoreinheit,
FIG. 3f ein sechstes Ausführungsbeispiel einer Kollimatoreinheit,
FIG. 4 ein Ausführungsbeispiel eines Steuersystems,
FIG. 5 ein Ausführungsbeispiel eines Mammographiesystems,
FIG. 6a ein erstes Ausführungsbeispiel einer Röntgenquelle,
FIG. 6b ein zweites Ausführungsbeispiel einer Röntgenquelle,
FIG. 6c ein drittes Ausführungsbeispiel einer Röntgenquelle.
FIG. 6d ein viertes Ausführungsbeispiel einer Röntgenquelle.

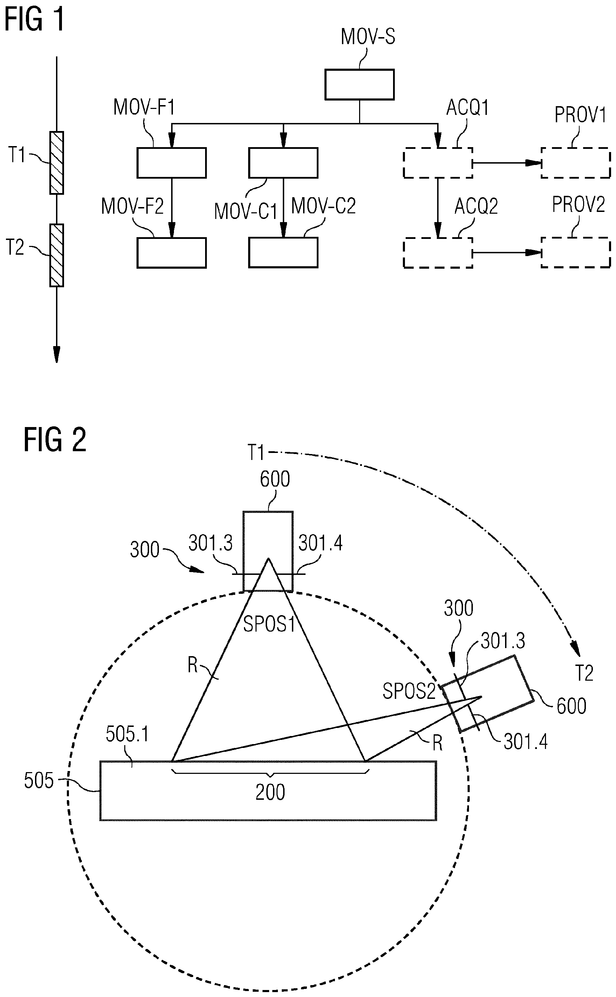

FIG. 1 zeigt ein erstes Ausführungsbeispiel eines Verfahrens zum Beleuchten eines Röntgenstrahlungsfeldes 200.

Auf der linken Seite der Abbildung ist ein Zeitstrahl abgebildet. Der Zeitstrahl verdeutlicht den zeitlichen Verlauf während einer Aufnahme. Die Aufnahme kann dabei wenigstens einen ersten und einen zweiten Belichtungszeitraum T1, T2 umfassen. Während dieser Belichtungszeiträume T1, T2 erfasst ein Röntgendetektor 505 Röntgenstrahlung R, die von einer Röntgenquelle 600 ausgesendet wird. Insbesondere kann eine Aufnahme mehr als zwei Belichtungszeiträume T1, T2 umfassen.

Der erste und der zweite Belichtungszeitraum T1, T2 können gleich oder verschieden lang sein. Der erste und der zweite Belichtungszeitraum T1, T2 können zeitlich direkt aneinander anschließen oder mit einer Pause zeitlich voneinander getrennt sein.

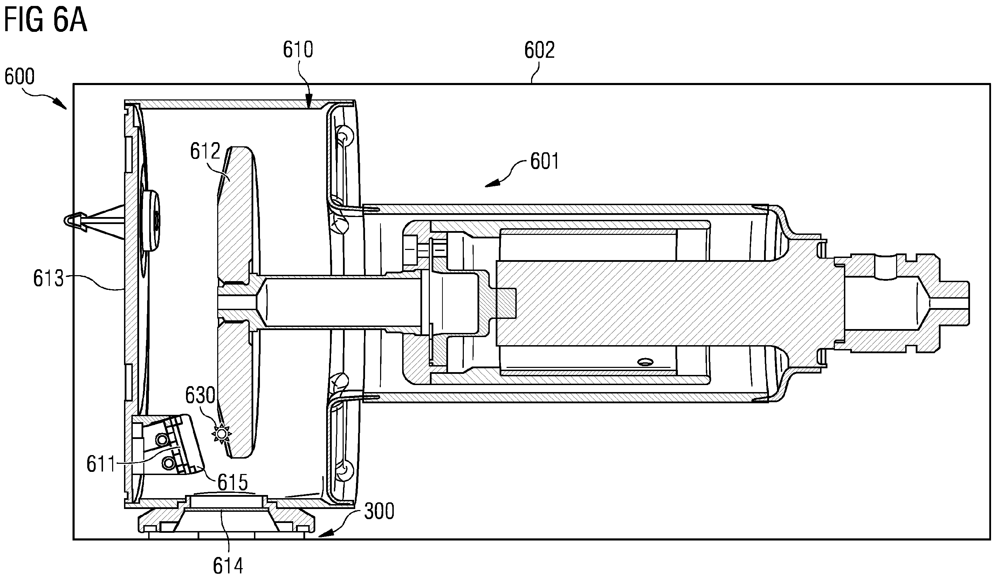

Die Röntgenquelle 600 kann insbesondere gemäß einer der Fig. **6a bis 6d** oder einer beliebigen Kombination ausgebildet sein.

Zwischen der Röntgenquelle 600 und dem Röntgendetektor kann ein Untersuchungsobjekt O angeordnet sein. Das Untersuchungsobjekt O kann dabei insbesondere ein Mensch bzw. ein Patient oder ein Tier oder ein Gegenstand sein. Wenn die Aufnahme eine Mammographieaufnahme umfasst, ist das Untersuchungsobjekt O typischerweise eine menschliche Brust.

Während der Aufnahme und insbesondere während des ersten und des zweiten Belichtungszeitraums T1, T2 wird die Röntgenquelle 600 in einem Verfahrensschritt eines Bewegens MOV-S der Röntgenquelle 600 relativ zu einem Röntgendetektor 505 bewegt. Insbesondere wird die Röntgenquelle 600 wenigstens während eines Teils des ersten Belichtungszeitraums T1 und während eines Teils des zweiten Belichtungszeitraums T2 bewegt. Insbesondere kann die Röntgenquelle 600 während des kompletten ersten Belichtungszeitraums T1 und während des kompletten zweiten Belichtungszeitraums T2 bewegt werden. In Ausführungen kann die Röntgenquelle 600 zwischen dem ersten und dem zweiten Belichtungszeitraum T1, T2 anhalten bzw. pausieren. Alternativ kann die Röntgenquelle 600 zwischen dem ersten und dem zweiten Belichtungszeitraum T1, T2 weiter bewegt werden.

Zwischen der Röntgenquelle 600 und dem Röntgendetektor 505 ist eine Kollimatoreinheit 300, 300', 300", 300‴, 300"", 300ʺʺ angeordnet. Die Kollimatoreinheit 300, 300', 300", 300‴, 300ʺʺ, 300‴ʺ ist dazu ausgebildet, die Röntgenstrahlung R auf das Röntgenstrahlungsfeld 200 zu begrenzen.

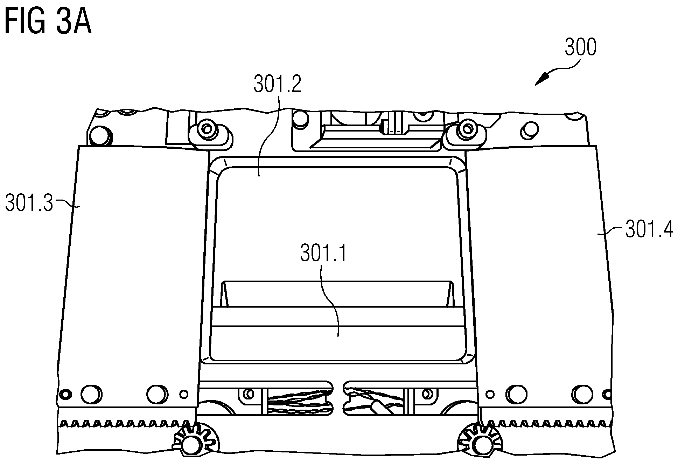

Die Kollimatoreinheit 300, 300', 300", 300‴, 300"", 300ʺʺ kann dabei insbesondere gemäß einer der Fig. **3a bis 3f** oder einer beliebigen Kombination daraus ausgebildet sein.

Während des ersten Belichtungszeitraums T1 wird in einem Verfahrensschritt eines Bewegens MOV-F1 eines Fokus' 630 der Röntgenstrahlung R relativ zu der Bewegung der Röntgenquelle 600 während des ersten Belichtungszeitraums T1 der Fokus 630 bewegt.

Gleichzeitig wird in einem Verfahrensschritt eines Bewegens MOV-C1 der Kollimatoreinheit 300, 300', 300", 300‴, 300"", 300ʺʺ während des ersten Belichtungszeitraums T1 die Kollimatoreinheit 300, 300', 300", 300‴, 300"", 300‴ʺ derart bewegt, dass das Röntgenstrahlungsfeld 200 konstant beleuchtet wird.

Konstant beleuchtet bedeutet dabei, dass das Röntgenstrahlungsfeld 200 trotz der Bewegung der Röntgenquelle 600 und der Bewegung des Fokus' 630 das Röntgenstrahlungsfeld 200 wenigstens teilweise ortsfest bleibt. Insbesondere kann auch eine Intensitätsverteilung innerhalb des Röntgenstrahlungsfeldes 200 während des ersten Belichtungszeitraums T1 wenigstens teilweise konstant bzw. gleich bleiben. "Wenigstens teilweise" bedeutet, dass kleinere Schwankungen und/oder eine Verschiebung bzw. Schwankung des Röntgenstrahlungsfeldes 200 während kleinerer Abschnitte des ersten Belichtungszeitraums T1 von der Erfindung umfasst sind.

In einem weiteren Verfahrensschritt eines Bewegens MOV-F2 des Fokus' 630 der Röntgenstrahlung R relativ zu der Bewegung der Röntgenquelle 600 während des zweiten Belichtungszeitraums T2 wird er Fokus 630 auch während des zweiten Belichtungszeitraums T2 bewegt.

Gleichzeitig wird auch hier in einem Verfahrensschritt eines Bewegens MOV-C2 der Kollimatoreinheit 300, 300', 300", 300‴, 300"", 300‴ʺ während des zweiten Belichtungszeitraums T2 die Kollimatoreinheit 300, 300', 300", 300‴, 300"", 300‴ʺ derart bewegt, dass das Röntgenstrahlungsfeld 200 wenigstens teilweise konstant beleuchtet wird.

Dabei nimmt die Kollimatoreinheit 300, 300', 300", 300‴, 300"", 300‴ʺ zu Beginn des ersten Belichtungszeitraums T1 eine erste Startposition SPOS1 ein und zu Beginn des zweiten Belichtungszeitraums T2 eine zweite Startposition SPOS2 ein. Die erste und die zweite Startposition SPOS1, SPOS2 sind verschieden. Die erste und die zweite Startposition SPOS1, SPOS2 hängen dabei insbesondere von der Position der Röntgenquelle 600 relativ zum Röntgendetektor 505 zu Beginn des entsprechenden Belichtungszeitraums T1, T2 ab.

In einem optionalen Verfahrensschritt eines Erfassens ACQ1 erfasst der Röntgendetektor 505 in dem ersten Belichtungszeitraum T1 die Röntgenstrahlung R und stellt diese in Form von ersten Bilddaten in einem Verfahrensschritt PROV1 bereit.

In einem weiteren optionalen Verfahrensschritt eines Erfassens ACQ2 erfasst der Röntgendetektor 505 in dem zweiten Belichtungszeitraum T2 die Röntgenstrahlung R und stellt diese in Form von zweiten Bilddaten in einem Verfahrensschritt PROV2 bereit.

Das Bereitstellen PROV1, PROV2 kann dabei ein Anzeigen oder ein Speichern der Bilddaten umfassen. Insbesondere können die ersten und die zweiten Bilddaten derart verarbeitet werden, dass eine dreidimensionale Darstellung bzw. Abbildung des Untersuchungsobjektes O bestimmt werden kann.

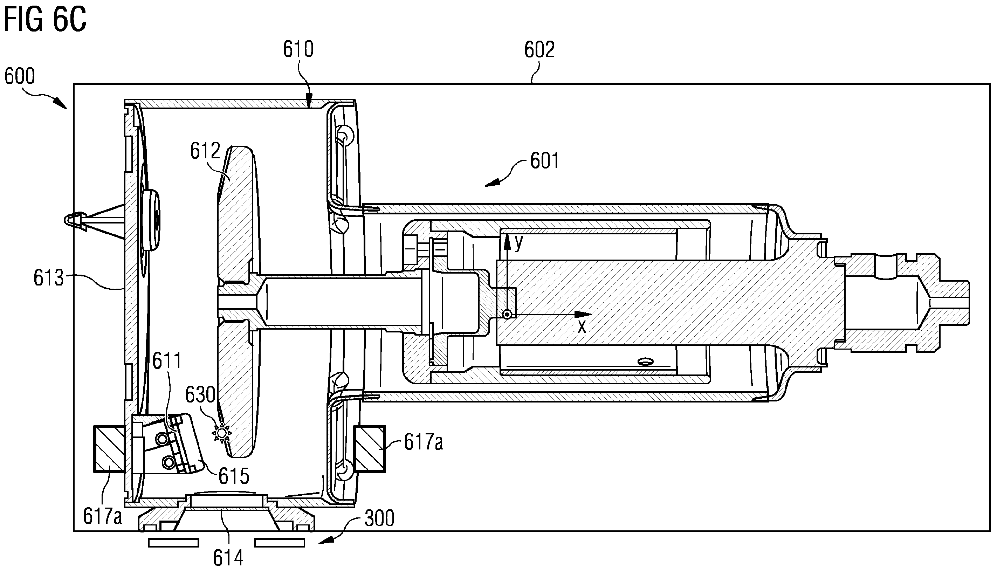

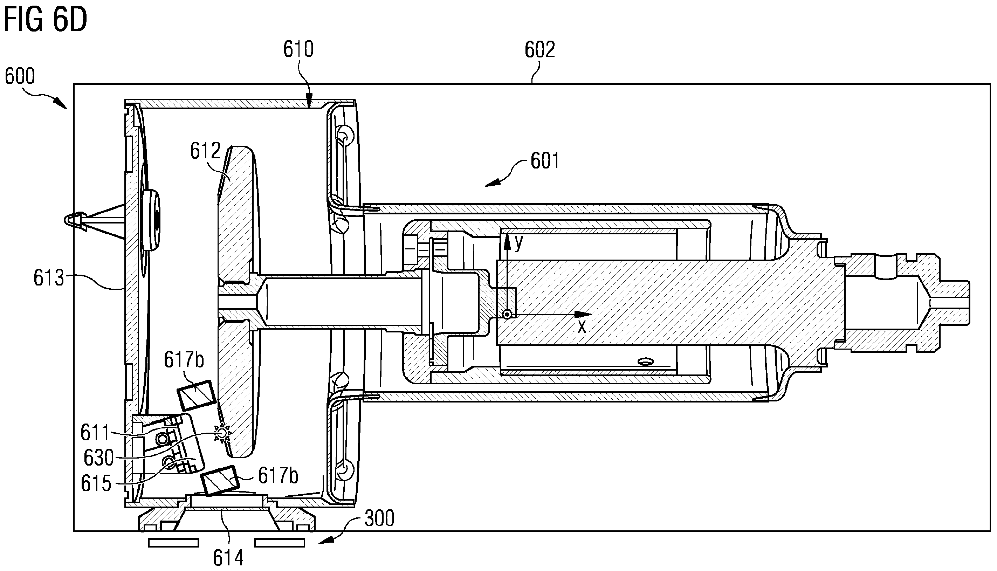

Optional ist die Bewegung des Fokus' 630 während des ersten und des zweiten Belichtungszeitraums T1, T2 derart ausgebildet, dass die Bewegung der Röntgenquelle 600 relativ zum Röntgendetektor 505 wenigstens teilweise derart kompensiert wird, dass der Fokus 630 aus Sicht des Röntgendetektors 505 wenigstens teilweise ortsfest ist. Dies kann insbesondere mittels Ablenksystemen 617a, 617b wie in den Fig. **6c** **und** **6d** beschrieben ermöglicht werden. "Wenigstens teilweise" bedeutet in diesem Fall, dass eine leichte Schwankung des Fokus' 630 aus Sicht des Röntgendetektors 505 während eines Teils des Belichtungszeitraums T1, T2 und/oder um einen Bereich, der kleiner ist, als der, der durch die Bewegung der Röntgenquelle 600 verursacht werden würde, auch von der erfinderischen Idee umfasst ist.

Optional wird die Kollimatoreinheit 300, 300', 300", 300‴, 300ʺʺ, 300‴ʺ während des ersten und/oder zweiten Belichtungszeitraums T1, T2 entgegengesetzt zu der Bewegung der Röntgenquelle 600 bewegt. Insbesondere wird die Kollimatoreinheit 300, 300', 300", 300‴, 300"", 300‴ʺ wenigstens während eines Teils des ersten und/oder während eines Teils des zweiten Belichtungszeitraums T1, T2 entgegengesetzt zu der Bewegung der Röntgenquelle 600 bewegt. Insbesondere wird die Kollimatoreinheit 300, 300', 300", 300‴, 300"", 300‴ʺ wenigstens teilweise bzw. zeitweise mit dem Fokus 630 mit bewegt. Insbesondere wird die Kollimatoreinheit 300, 300', 300", 300‴, 300"", 300‴ʺ während eines Belichtungszeitraums T1, T2 entgegengesetzt zu der Bewegung der Röntgenquelle 600 bewegt.

Optional ist das Röntgenstrahlungsfeld 200 wie in **Fig. 2** dargestellt auf einer Oberfläche 505.1 des Röntgendetektors 500 angeordnet. Insbesondere kann das Röntgenstrahlungsfeld 200 der gesamten aktiven Oberfläche 505.1 des Röntgendetektors 505 entsprechen und beispielsweise durch seine Kanten begrenzt werden. Alternativ kann das Röntgenstrahlungsfeld 200 in Bereichen größer als die aktive Fläche des Röntgendetektors 505 sein und über diesen hinaus ragen. Alternativ kann das Röntgenstrahlungsfeld 200 kleiner als die aktive Fläche des Röntgendetektors 505 sein und auf das Untersuchungsobjekt O eingeschränkt sein.

Alternativ kann das Röntgenstrahlungsfeld 200 in einer Ebene innerhalb des Untersuchungsobjektes O angeordnet sein. Die Ebene kann parallel zu der Oberfläche 505.1 des Röntgendetektors 505 ausgerichtet sein. Alternativ kann die Ebene in einem Winkel zu der Oberfläche 505.1 des Röntgendetektors 505 ausgerichtet sein. Die Ebene kann beispielsweise auf einer Oberfläche des Untersuchungsobjektes O angeordnet sein. Alternativ kann die Ebene in einer mittleren Schicht des Untersuchungsobjektes O aus Sicht des Röntgendetektors angeordnet sein.

Optional kann die Kollimatoreinheit 300, 300', 300", 300"', 300"", 300‴ʺ basierend auf der Bewegung des Fokus' 630 bewegt werden. Insbesondere kann die Kollimatoreinheit 300, 300', 300", 300‴, 300ʺʺ, 300‴ʺ wenigstens teilweise während des ersten und/oder zweiten Belichtungszeitraums T1, T2 mit dem Fokus 630 mit bewegt werden.

Optional kann die Kollimatoreinheit 300, 300', 300", 300‴, 300ʺʺ, 300‴ʺ außerdem basierend auf der Bewegung der Röntgenquelle 600 während des ersten und/oder zweiten Belichtungszeitraums T1, T2 bewegt werden.

Optional hängt die erste Startposition SPOS1 der Kollimatoreinheit 300, 300', 300", 300‴, 300"", 300‴ʺ von einer Position der Röntgenquelle 600 relativ zum Röntgendetektor 505 zu Beginn des ersten Belichtungszeitraums T1 ab. Optional hängt außerdem die zweite Startposition SPOS2 der Kollimatoreinheit 300, 300', 300", 300‴, 300"", 300‴ʺ von einer Position der Röntgenquelle 600 relativ zum Röntgendetektor 505 zu Beginn des zweiten Belichtungszeitraums T2 ab. Insbesondere wird bei der Wahl bzw. beim Bestimmen der ersten bzw. zweiten Startposition SPOS1, SPOS2 die relative Position zwischen Röntgenquelle 600 und Röntgendetektor 505 berücksichtigt. Insbesondere wird die relative Position derart berücksichtigt, dass vorteilhafterweise für jede Position der Röntgenquelle 600 relativ zum Röntgendetektor 505 zu Beginn eines Belichtungszeitraums T1, T2 immer konstant dasselbe Röntgenstrahlungsfeld 200 beleuchtet wird.

Optional umfasst die Kollimatoreinheit 300, 300', 300", 300‴, 300"", 300‴ʺ wenigstens zwei Lamellen 301.1, 301.1ʺʺ, 301.2, 301.2', 301.2"", 301.3, 301.3", 301.3‴, 301.3ʺʺ, 301.4, 304.4", 301.4"', 301.4"", 301a, 301b, 301c, 301d. Dabei gibt die erste Startposition SPOS1 wenigstens eine Startposition wenigstens einer der Lamellen 301.1, ..., 301d relativ zu der Röntgenquelle 600 zu Beginn des ersten Belichtungszeitraums T1 vor. Dabei gibt die zweite Startposition SPOS2 wenigstens eine Startposition wenigstens einer der Lamellen 301.1, ..., 301d relativ zu der Röntgenquelle 600 zu Beginn des zweiten Belichtungszeitraums T2 vor. Mit anderen Worten kann die erste bzw. zweite Startposition SPOS1, SPOS2 auch lediglich eine Position für nur einen Teil der Kollimatoreinheit 300, 300', 300", 300"', 300"", 300‴ʺ vorgeben. In Ausführungen kann beispielsweise die Kollimatoreinheit 300, 300', 300", 300‴, 300ʺʺ, 300‴ʺ vier Lamellen 301.1, ..., 301d umfassen. Die erste und die zweite Startposition können sich dann in Ausführungen lediglich durch die Startposition jeweils einer einzigen Lamelle 301.1, ..., 301d unterscheiden. Alternativ können sich die erste und die zweite Startposition SPOS1, SPOS2 durch die Startpositionen von mehr als einer Lamelle 301.1, ..., 301d unterscheiden.

Optional umfasst die Kollimatoreinheit 300, 300', 300", 300‴, 300"", 300‴ʺ wenigstens zwei Lamellen 301.1, ..., 301d. Dabei gibt die erste Startposition SPOS1 wenigstens einen Abstand der beiden Lamellen 301.1, ..., 301d zueinander zu Beginn des ersten Belichtungszeitraums T1 vor. Dabei gibt die zweite Startposition SPOS2 wenigstens einen Abstand der beiden Lamellen 301.1, ..., 301d zueinander zu Beginn des zweiten Belichtungszeitraums T2 vor. Da sich die Röntgenquelle 600 zwischen dem Beginn des ersten Belichtungszeitraums T1 und dem Beginn des zweiten Belichtungszeitraums T2 bewegt, kann es notwendig sein, dass sich die Abstände der Lamellen 301.1, ..., 301d zu Beginn der beiden Belichtungszeiträume T1, T2 unterscheiden, damit jeweils ein konstantes, insbesondere ortsfestes Röntgenstrahlungsfeld 200 beleuchtet wird.

Optional ist die Bewegung der Röntgenquelle 600 während des ersten und des zweiten Belichtungszeitraums T1, T2 und insbesondere während der gesamten Aufnahme kontinuierlich. Insbesondere kann die Bewegung der Röntgenquelle 600 als eine bogenförmige Bewegung relativ zu dem Röntgendetektor 505 ausgebildet sein.

**FIG. 2** zeigt ein zweites Ausführungsbeispiel eines Verfahrens zum Beleuchten eines Röntgenstrahlungsfeldes 200.

Das Verfahren kann prinzipiell gemäß der Beschreibung zu **Fig. 1** ausgebildet sein.

Die Abbildung zeigt beispielhaft die Position der Röntgenquelle 600 zu Beginn des ersten und des zweiten Belichtungszeitrums T1, T2. In der Zwischenzeit wurde die Röntgenquelle 600 entlang eines Kreisbogens relativ zu dem Röntgendetektor 505 bewegt.

In diesem Ausführungsbeispiel ist das Röntgenstrahlungsfeld 200 auf der Oberfläche 505.1 des Röntgendetektors 500 definiert. Alternativ kann das Röntgenstrahlungsfeld 200 in einer Ebene eines Untersuchungsobjektes O, welches zwischen der Röntgenquelle 600 und dem Röntgendetektor 505 positioniert sein kann, ausgebildet bzw. angeordnet sein. Aus Gründen der Übersichtlichkeit wurde hier auf die Abbildung des Untersuchungsobjektes O verzichtet.

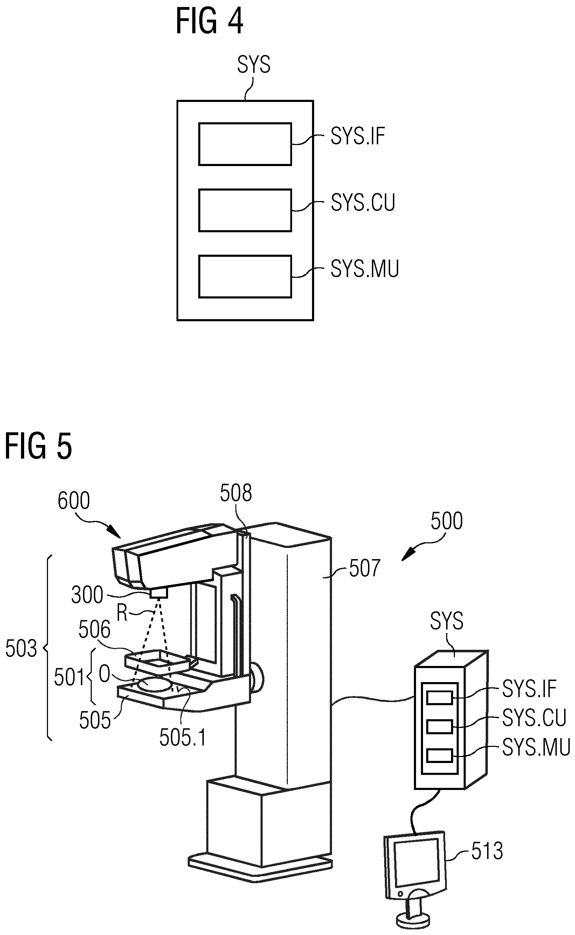

In dem dargestellten Ausführungsbeispiel ist die Kollimatoreinheit 300 innerhalb der Röntgenquelle 600, insbesondere innerhalb des Eintanks 602 angeordnet. Alternativ kann die Kollimatoreinheit 300 auch in einer der anderen, in der Beschreibung zu **Fig. 5** genannten Positionen angeordnet sein.

Die Kollimatoreinheit 300 kann insbesondere auch gemäß einem alternativen Ausführungsbeispiel wie in den **Fig. 3a bis 3f** beschrieben oder einer beliebigen Kombination ausgebildet sein. Insbesondere kann die Kollimatoreinheit 300 zwei Sub-Einheiten umfassen, die an unterschiedlichen Positionen angeordnet sind. Insbesondere können die Sub-Einheiten auch unterschiedlich ausgebildet sein.

In dem Ausführungsbeispiel sind beispielhaft zwei Lamellen 301.3, 301.4 der Kollimatoreinheit 300 abgebildet, die einen Abstand zueinander aufweisen, der das Röntgenstrahlungsfeld 200 definiert.

Es sind die erste und die zweite Startposition SPOS1, SPOS2 der Kollimatoreinheit 300 zu Beginn des ersten bzw. zweiten Belichtungszeitraums T1, T2 dargestellt. Es wird deutlich, dass der Abstand der beiden dargestellten Lamellen 301.3, 301.4 der Kollimatoreinheit 300 in der ersten Startposition SPOS1 größer ist als in der zweiten Startposition SPOS2. Außerdem sind die Lamellen 301.3, 301.4 in der zweiten Startposition SPOS2 weiter nach rechts verschoben als in der ersten Starposition SPOS1.

**FIG. 3a** zeigt ein erstes Ausführungsbeispiel einer Kollimatoreinheit 300.

Die Kollimatoreinheit 300 ist insbesondere dazu ausgebildet, in dem Verfahren wie bezüglich der **Fig. 1** **und/oder 2** beschrieben verwendet zu werden.

Die Kollimatoreinheit 300 ist insbesondere zur Verwendung in einem Mammographiesystem 500 ausgebildet.

Die Kollimatoreinheit 300 umfasst in dem Ausführungsbeispiel vier Lamellen. Insbesondere umfasst die Kollimatoreinheit 300 zwei seitliche Lamellen 301.3, 301.4, eine Lamelle 301.1, die nahe an einer Brustwand eines Untersuchungsobjektes O, insbesondere eines Patienten, angeordnet ist, und eine Lamelle 301.2, die dieser gegenüber angeordnet ist.

Die Lamellen 301.1, 301.2, 301.3, 301.4 können dabei in Ausführungen der Erfindung unabhängig voneinander bewegt werden. Dabei bedeutet unabhängig voneinander, dass sie mit unterschiedlichen Geschwindigkeiten und/oder in unterschiedliche Richtungen und/oder asynchron und/oder synchron zueinander bewegt werden können. Dafür können die vier Lamellen 301.1, 301.2, 301.3, 301.4 jeweils an verschiedenen Linearverstelleinheiten angeordnet sein. Alternativ können wenigstens zwei Lamellen 301.1, 301.2, 301.3, 301.4 an einer gemeinsamen Linearverstelleinheit angeordnet sein. Diese Lamellen 301.1, 301.2, 301.3, 301.4 können dann gekoppelt also synchron bewegt werden.

In Ausführungen der Erfindung kann wenigstens eine der Lamellen 301.1, 301.2, 301.3, 301.4 rotierbar ausgebildet sein. Insbesondere kann die brustwand abgewandt angeordnete Lamelle 301.1 rotierbar ausgebildet sein. Dabei ist die Lamelle 301.1, 301.2, 301.3, 301.4 vorteilhafterweise um eine Achse senkrecht auf die Lamellenfläche rotierbar.

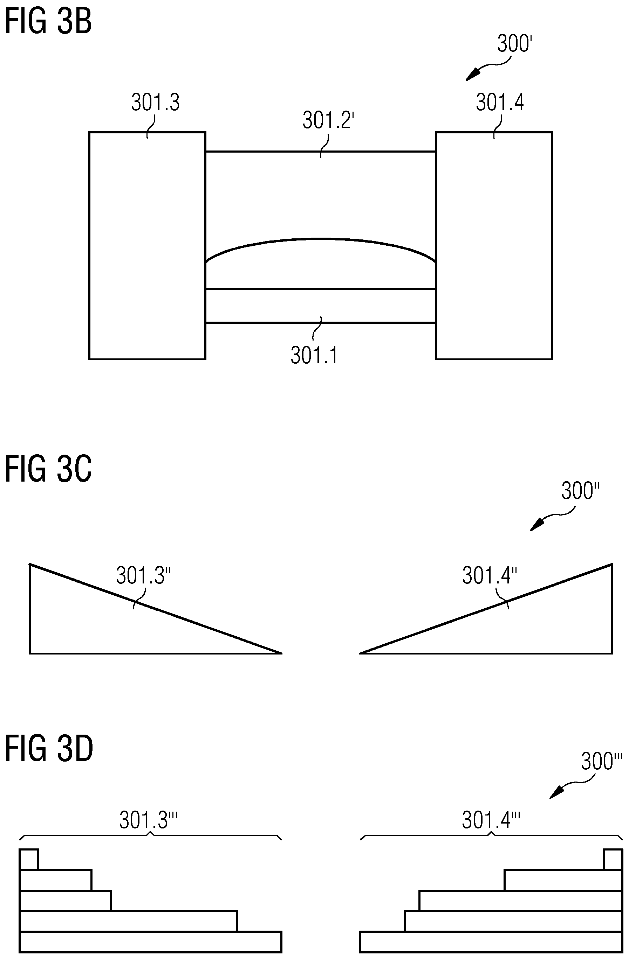

**FIG. 3b** zeigt ein zweites Ausführungsbeispiel einer Kollimatoreinheit 300'.

Die Kollimatoreinheit 300' ist insbesondere dazu ausgebildet, in dem Verfahren wie bezüglich der **Fig. 1** **und/oder 2** beschrieben verwendet zu werden.

Die Kollimatoreinheit 300' ist insbesondere zur Verwendung in einem Mammographiesystem 500 ausgebildet.

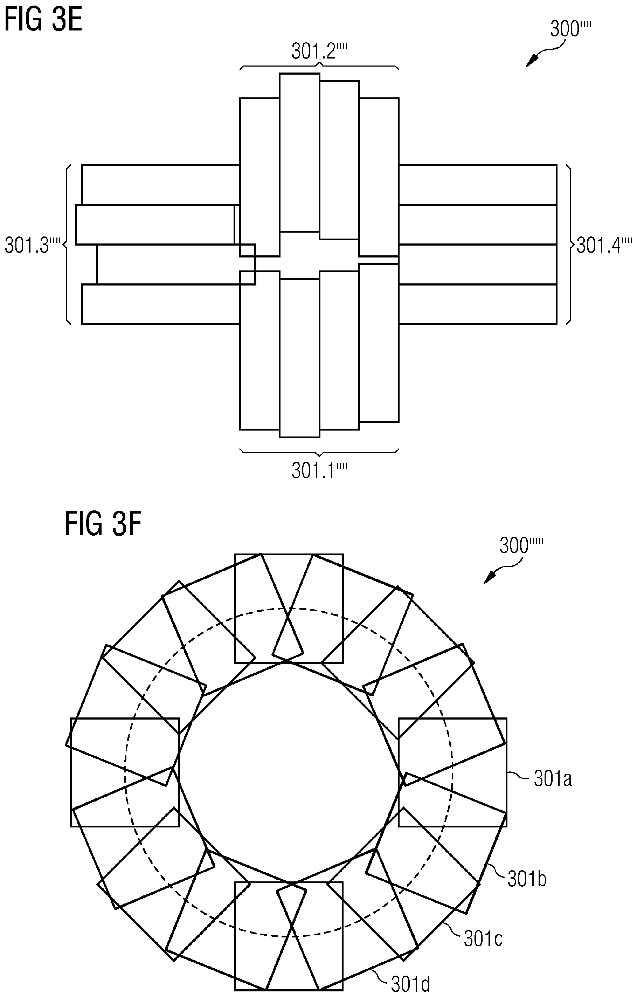

Die Kollimatoreinheit 300' des zweiten Ausführungsbeispiels kann beliebig mit der Kollimatoreinheit 300 des ersten Ausführungsbeispiels kombiniert werden. Insbesondere kann die allgemeine Beschreibung zu **Fig. 3** auf das zweite Ausführungsbeispiel übertragen werden.

In dem zweiten Ausführungsbeispiel weist eine der Lamellen 301.2' eine gekrümmte Kante auf. In dem Ausführungsbeispiel ist die Kante der der brustwandseitig angeordneten Lamelle 301.1 gegenüberliegende Lamelle 301.2' gekrümmt. Alternativ kann auch jede andere Lamelle 301.1, 301.2', 301.3, 301.4 eine gekrümmte Kante aufweisen. Insbesondere kann eine beliebige Anzahl an Lamellen 301.1, 301.2, 301.3, 301.4 gekrümmte Kanten aufweisen. Die Krümmung kann jede beliebige Form aufweisen und insbesondere an die Anwendung angepasst sein.

**FIG. 3c** zeigt ein drittes Ausführungsbeispiel einer Kollimatoreinheit 300".

Die Kollimatoreinheit 300" ist insbesondere dazu ausgebildet, in dem Verfahren wie bezüglich der **Fig. 1** **und/oder 2** beschrieben verwendet zu werden.

Die Kollimatoreinheit 300" ist insbesondere zur Verwendung in einem Mammographiesystem 500 ausgebildet.

Die Kollimatoreinheit 300" des dritten Ausführungsbeispiels kann beliebig mit der Kollimatoreinheit 300, 300' des ersten Ausführungsbeispiels und/oder des zweiten Ausführungsbeispiels kombiniert werden. Insbesondere kann die allgemeine Beschreibung zu **Fig. 3** auf das dritte Ausführungsbeispiel übertragen werden.

In der Abbildung ist ein Querschnitt durch die beiden seitlichen Lamellen 301.3", 301.4" dargestellt. In Ausführungen der Erfindung kann der Querschnitt der anderen Lamellen 301.1, 301.2 analog ausgebildet sein.

Der Querschnitt der beiden dargestellten Lamellen 301.3", 301.4" zeigt eine variable Dicke der Lamellen 301.3", 301.4". In dem dargestellten Ausführungsbeispiel ist der Querschnitt keilförmig ausgebildet mit zunehmender Dicke zum Rand hin. Alternative Ausführungen, insbesondere eine stufenförmige Variation der Dicke sind denkbar.

Insbesondere kann die Dicke der Lamelle 301.3", 301.4" derart ausgebildet sein, dass eine Inhomogenität der Röntgenstrahlung R in dem Röntgenstrahlungsfeld 200 ausgeglichen wird.

**FIG. 3d** zeigt ein viertes Ausführungsbeispiel einer Kollimatoreinheit 300‴.

Die Kollimatoreinheit 300‴ ist insbesondere dazu ausgebildet, in dem Verfahren wie bezüglich der **Fig. 1** **und/oder 2** beschrieben verwendet zu werden.

Die Kollimatoreinheit 300‴ ist insbesondere zur Verwendung in einem Mammographiesystem 500 ausgebildet.

Die Kollimatoreinheit 300‴ des vierten Ausführungsbeispiels kann beliebig mit der Kollimatoreinheit 300, 300', 300" des ersten Ausführungsbeispiels und/oder des zweiten Ausführungsbeispiels und/oder des dritten Ausführungsbeispiels kombiniert werden. Insbesondere kann die allgemeine Beschreibung zu **Fig. 3** auf das vierte Ausführungsbeispiel übertragen werden.

In der Abbildung ist ein Querschnitt durch die beiden seitlichen Lamellen 301.3"', 301.4‴ dargestellt. In Ausführungen der Erfindung kann der Querschnitt der anderen Lamellen 301.1, 301.2 analog ausgebildet sein.

Die seitlichen Lamellen 301.3"', 301.4‴ werden durch eine Vielzahl von zueinander verschiebbaren und/oder auswechselbaren Platten ausgebildet. Durch Verschieben und/oder Austauschen der Platten kann die Verteilung der Dicke der Lamellen 301.3"', 301.4‴ variiert werden und an die Inhomogenitäten der Röntgenstrahlung R in dem Röntgenstrahlungsfeld 200 angepasst werden.

**FIG. 3e** zeigt ein fünftes Ausführungsbeispiel einer Kollimatoreinheit 300"".

Die Kollimatoreinheit 300ʺʺ ist insbesondere dazu ausgebildet, in dem Verfahren wie bezüglich der **Fig. 1** **und/oder 2** beschrieben verwendet zu werden.

Die Kollimatoreinheit 300ʺʺ ist insbesondere zur Verwendung in einem Mammographiesystem 500 ausgebildet.

Die Kollimatoreinheit 300ʺʺ des fünften Ausführungsbeispiels kann beliebig mit der Kollimatoreinheit 300, 300', 300", 300‴ des ersten Ausführungsbeispiels und/oder des zweiten Ausführungsbeispiels und/oder des dritten Ausführungsbeispiels und/oder des vierten Ausführungsbeispiels kombiniert werden. Insbesondere kann die allgemeine Beschreibung zu **Fig. 3** auf das fünfte Ausführungsbeispiel übertragen werden.

Die Lamellen 301.1ʺʺ, 301.2"", 301.3"", 301.4ʺʺ des fünften Ausführungsbeispiels umfassen jeweils eine Mehrzahl von Sub-Lamellen, die zueinander verschiebbar ausgebildet sind. Dadurch können die Kanten der Lamellen 301.1"", 301.2ʺʺ, 301.3"", 301.4ʺʺ beliebig an das Untersuchungsobjekt O und/oder das Röntgenstrahlungsfeld 200 angepasst werden.

**FIG. 3f** zeigt ein sechstes Ausführungsbeispiel einer Kollimatoreinheit 300‴ʺ.

Die Kollimatoreinheit 300ʺʺ ist insbesondere dazu ausgebildet, in dem Verfahren wie bezüglich der **Fig. 1** **und/oder 2** beschrieben verwendet zu werden.

Die Kollimatoreinheit 300‴ʺ ist insbesondere zur Verwendung in einem Mammographiesystem 500 ausgebildet.

Die Kollimatoreinheit 300‴ʺ des sechsten Ausführungsbeispiels kann beliebig mit der Kollimatoreinheit 300", 300"'des dritten Ausführungsbeispiels und/oder des vierten Ausführungsbeispiels kombiniert werden. Insbesondere kann die allgemeine Beschreibung zu **Fig. 3** auf das sechste Ausführungsbeispiel übertragen werden.

Die Kollimatoreinheit 300‴ʺ umfasst eine Vielzahl von Lamellen 301a, 301b, 301c, 301d, die insbesondere in ihrer Gesamtheit einen Kreis bilden können. Insbesondere kann durch Verschieben der Lamellen 301a, 301b, 301c, 301d die Größe der Öffnung des Kreises und damit das Röntgenstrahlungsfeld 200 variiert werden.

In Ausführungen der Erfindung können die Lamellen 301a, 301b, 301c, 301d auch derart relativ zueinander verschoben werden, dass die Öffnung nicht mehr kreisförmig, sondern beispielsweise oval ausgebildet ist.

**FIG. 4** zeigt ein Ausführungsbeispiel eines Steuersystems SYS.

Das dargestellte Steuersystem SYS ist dazu ausgebildet, ein erfindungsgemäßes Verfahren zum Beleuchten eines Röntgenstrahlungsfeldes 200 auszuführen. Das Steuersystems SYS umfasst eine Schnittstelle SYS.IF, eine Recheneinheit SYS.CU und eine Speichereinheit SYS.MU.

Das Steuersystem SYS kann insbesondere ein Computer, ein Mikrocontroller oder ein integrierter Schaltkreis (integrated circuit, IC) sein. Alternativ kann das Steuersystem SYS ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Das Steuersystem SYS kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization").

Die Schnittstelle SYS.IF kann eine Hardware- oder Software-Schnittstelle sein (beispielsweise ein PCI-Bus, CAN-Bus, USB oder Firewire). Die Recheneinheit SYS.CU kann Hardware- und/oder Software-Bestandteile umfassen, beispielsweise einen Mikroprozessor oder einen sogenannten FPGA (Field Programmable Gate Way). Die Speichereinheit SYS.MU kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle SYS.IF kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle SYS.IF als eine Mehrzahl an Schnittstellen SYS.IF ausgebildet sein. Die Recheneinheit SYS.CU kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit SYS.CU als eine Mehrzahl an Recheneinheiten SYS.CU ausgebildet sein.

**FIG. 5** zeigt ein Ausführungsbeispiel eines Mammographiesystems 500.

Das Mammographiesystem 500 ist beispielhaft und grob schematisch dargestellt. Das Mammographiesystem 500 kann insbesondere für eine Tomosynthese ausgebildet sein. Relative Richtungen wie "oben", "unten" usw. beziehen sich auf das Mammographiesystem 500, das für den bestimmungsgemäßen Betrieb eingerichtet wurde. Das Mammographiesystem 500 hat eine stehende Säule 507 und eine Quellen-Detektor-Anordnung 503, die wiederum eine Röntgenquelle 600 und einen Röntgendetektor 505 mit einer Oberfläche bzw. Abdeckung 505.1 umfasst. Die Röntgenquelle 600 ist insbesondere gemäß der Beschreibung zu einer der **Fig. 6a bis 6d** oder einer beliebigen Kombination dieser Figuren ausgebildet. Die stehende Säule 507 ist am Boden montiert. Die Quellen-Detektor-Anordnung 503 kann beweglich mit der stehenden Säule 507 verbunden werden, so dass die Höhe der Oberfläche 505.1 des Röntgendetektors 505 auf eine Brusthöhe eines Untersuchungsobjektes, insbesondere eines Patienten eingestellt werden kann.

Das Untersuchungsobjekt O, insbesondere eine Brust des Patienten (hier schematisch dargestellt) ist für eine Untersuchung auf der Oberfläche 505.1 des Röntgendetektors 505 positioniert. Die Oberfläche 505.1 bildet hierbei eine zweite Kompressionsplatte aus. Über der Brust O und der Oberfläche 505.1 ist eine Kompressionsplatte 506 angeordnet, die beweglich bzw. verfahrbar mit der Quellen Detektor-Anordnung 503 verbunden ist. Für die Untersuchung wird die Brust O komprimiert und gleichzeitig fixiert, indem die Kompressionsplatte 506 darauf abgesenkt wird, so dass Druck auf die Brust O zwischen Kompressionsplatte 506 und Abdeckung 505.1 ausgeübt wird. Alternativ kann für die Kompression anstelle der Abdeckung 505.1 des Röntgendetektors 505 eine zweite Kompressionsplatte verwendet werden, sodass die Brust O zwischen zwei Kompressionsplatten komprimiert wird. Die beiden Kompressionsplatten oder die Kompressionsplatte 506 und die Oberfläche 505.1 des Röntgendetektors 505 werden von einer Kompressionseinheit 501 umfasst. Die Kompressionseinheit 501 kann außerdem noch eine Verfahreinheit umfassen, um die Kompressionsplatte 506 bzw. die Kompressionsplatten zum Komprimieren zu verfahren.

Die Röntgenquelle 600 ist gegenüber dem Röntgendetektor 505 so angeordnet, dass der Röntgendetektor 505 von der Röntgenquelle 600 emittierte Röntgenstrahlung R detektiert, nachdem zumindest ein Teil der Röntgenstrahlung R die Brust O passiert hat.

Die Röntgenquelle 600 kann in Ausführungen relativ zum Röntgendetektor 505 mittels eines Dreharms 508 beispielsweise in einem Bereich von ± 25° um eine Grundposition, in der der

Dreharm 508 senkrecht zur Oberfläche 505.1 des Röntgendetektors 505 steht, bewegbar bzw. rotierbar bzw. verschwenkbar ausgebildet sein.

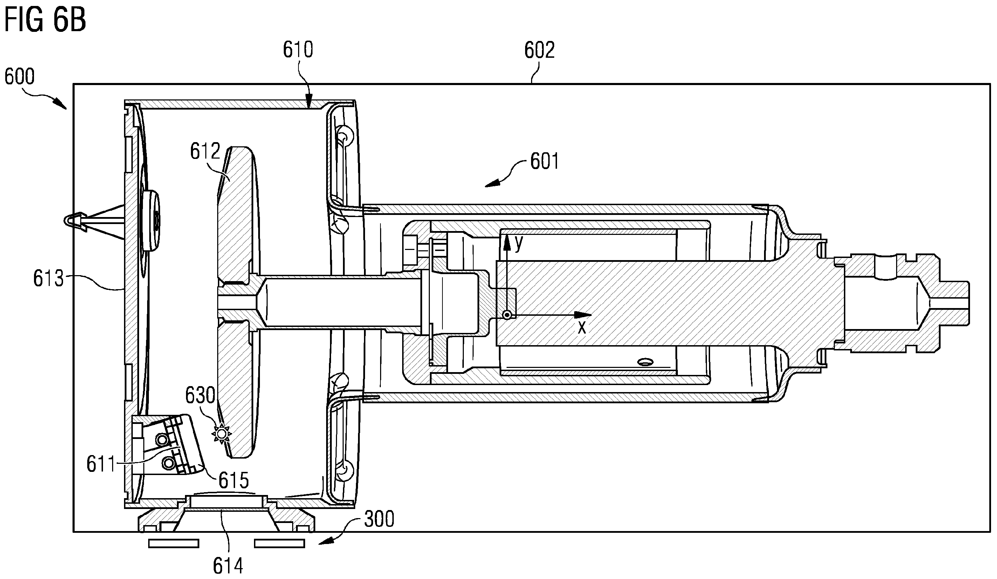

Das Mammographiesystem 500 umfasst außerdem eine Kollimatoreinheit 300, 300', 300", 300"', 300ʺʺ, 300‴ʺ. Die Kollimatoreinheit 300, 300', 300", 300‴, 300"", 300‴ʺ kann insbesondere gemäß der Beschreibung zu den **Fig. 3a bis 3f** ausgebildet sein. Die Kollimatoreinheit 300, 300', 300", 300‴, 300ʺʺ, 300‴ʺ kann dabei innerhalb eines Eintanks 602 der Röntgenquelle 600 (siehe Fig. 6a) und/oder vor dem Röntgenaustrittsfenster 614 der Röntgenquelle 600 (siehe Fig. 6b) und/oder auf der Kompressionsplatte 506 und/oder auf der Oberfläche 505.1 des Röntgendetektors 505 angeordnet sein. Alternativ oder zusätzlich kann die Kollimatoreinheit 300, 300', 300", 300"', 300ʺʺ, 300‴ʺ auf einer Achse zwischen der Röntgenquelle 600 und der Oberfläche 505.1 des Röntgendetektors 505 verfahrbar angeordnet sein. Durch das Verfahren der Kollimatoreinheit 300, 300', 300", 300‴, 300"", 300‴ʺ kann ein Abstand zwischen der Kollimatoreinheit 300, 300', 300", 300‴, 300"", 300‴ʺ und der Oberfläche 505.1 des Röntgendetektors 505 variiert werden.

In Ausführungen der Erfindung kann das Mammographiesystem 500 eine Kollimatoreinheit 300, 300', 300", 300‴, 300"", 300‴ʺ umfassen, die wenigstens zwei Sub-Einheiten umfasst. Jede der Sub-Einheiten kann an einer der zuvor beschriebenen Positionen des Mammographiesystems 500 angeordnet sein. Insbesondere sind die beiden Sub-Einheiten derart angeordnet, dass die Abstände der einzelnen Sub-Einheiten zu der Oberfläche 505.1 des Röntgendetektors 505 verschieden sind. Jede der Sub-Einheiten kann insbesondere wie eines der Ausführungsbeispiele gemäß der **Fig. 3a bis 3f** oder einer Kombination davon ausgebildet sein.

Das Mammographiesystem 500 kann insbesondere ein Steuersystem SYS mit einer Schnittstelle SYS.IF, einer Recheneinheit SYS.CU und einer Speichereinheit SYS.MU umfassen. Das Steuersystem SYS kann insbesondere mit einem Terminal 513 verbunden sein, das beispielsweise über eine Benutzeroberfläche oder Anzeigeeinheit verfügt, über die ein Benutzer Befehle an das Mammographiesystem 500 übermitteln oder Messergebnisse, beispielsweise die erfassten Projektionsaufnahmen bzw. Röntgenaufnahmen, abrufen kann. Das Steuersystem SYS kann sich im selben Raum wie das Mammographiesystem 500 befinden, es kann sich aber auch in einem angrenzenden Kontrollraum oder in einer noch größeren räumlichen Entfernung befinden.

Das Steuersystem SYS ist dabei insbesondere gemäß der Beschreibung zu **Fig. 4** ausgebildet.

**FIG. 6a** zeigt ein erstes Ausführungsbeispiel einer Röntgenquelle 600 mit einer Kollimatoreinheit 300 im Eintank.

Die Röntgenquelle 600 umfasst eine Röntgeneinheit 601. Die Röntgeneinheit 601 ist von einem Vakuumgehäuse 610 umschlossen. Die Röntgeneinheit 601 umfasst eine Kathode 611 und eine Anode 612. Die Kathode 611 und die Anode 612 sind innerhalb des Vakuumgehäuses 610 angeordnet. Das Vakuumgehäuse 610 kann durch einen Kathodendeckel 613 vakuumdicht abgeschlossen sein. Die Kathode 611 emittiert Elektronen, die wenigstens teilweise in Richtung der Anode 612 beschleunigt werden und beim Auftreffen in einem Fokus 630, auch als Brennfleck bezeichnet, im Material der Anode 612 Röntgenstrahlung R erzeugen. Die beschleunigten Elektronen können als Elektronenstrahl oder Elektronenwolke bezeichnet werden. Die Anode 612 ist als Drehanode ausgeführt, so dass der Fokus 630 eine Brennbahn bildet. Die erzeugte Röntgenstrahlung R tritt als Strahlenbündel über ein Röntgenaustrittsfenster bzw. Austrittsfenster bzw. Strahlenaustrittsfenster 614 aus dem Vakuumgehäuse 610 aus. Die Röntgenstrahlung R kann durch eine Öffnung, einem Röntgenaustrittsfenster 614 der Röntgenquelle 600 austreten, die von einem Fenster oder einem Filter bedeckt sein kann, um unerwünschte Strahlung zu blockieren oder zu reduzieren.

Die als Drehanode ausgebildete Anode 612 kann als rotationssymmetrischer Körper ausgebildet sein, der mit einem Anodenrohr und einem Wärmespeicher koppelbar oder gekoppelt ist und eine Brennbahn zur Erzeugung von Röntgenstrahlung aufweist. Der rotationssymmetrische Körper ist insbesondere eine Röntgenanodentasse bzw. ein Röntgenanodenteller. Typischerweise weist der Körper Molybdän auf und/oder besteht aus Molybdän oder einer Molybdän-basierten Legierung (z.B. TZM oder MHC). Typischerweise weist die Brennbahn Wolfram auf und/oder besteht aus Wolfram oder einer Wolfram-basierten Legierung (z.B. Wolfram-Rhenium). Der Körper ist insbesondere ein Trägerkörper für die Brennbahn. Die Brennbahn kann vom Trägerkörper strukturell, insbesondere materialtechnisch, verschieden sein. Die Anode 612 ist insbesondere für eine medizinische oder nichtmedizinische Bildgebung in einem kV-Bereich zwischen 10 und 200 kV geeignet. Der kV-Bereich beschreibt insbesondere eine maximale Beschleunigungsspannung, welche eine maximale Photonenenergie der generierbaren Röntgenstrahlung vorgibt. Die Röntgenstrahlung wird von der Oberseite der Anode abgestrahlt, die vorzugsweise eine geeignete Form und ein geeignetes Material aufweist, um eine hohe Röntgenausbeute zu erzielen.

Die als Drehanode ausgebildete Anode 612 ist grundsätzlich dazu geeignet, um um eine Rotationsachse drehgelagert zu sein. Für die Drehlagerung ist die Rotationsachse typischerweise mit dem Anodenrohr gekoppelt. Das Anodenrohr kann hohl oder in einer anderen Ausgestaltung aus Vollmaterial sein. Das Anodenrohr kann mit einem Rotor eines Drehantriebs gekoppelt sein. Das Anodenrohr ist typischerweise rotationssymmetrisch um die Symmetrieachse des Körpers ausgebildet.

In einer Ausführungsform weist die Anode 612 einen Wärmespeicher auf, wobei die Unterseite des Körpers in einem peripheren Abschnitt mit dem Wärmespeicher wärmeleitend gekoppelt ist, wobei der Wärmespeicher eine höhere Wärmeleitfähigkeit und/oder eine höhere Wärmekapazität aufweist als der Körper. Der Wärmespeicher besteht typischerweise aus Grafit. Die Kopplung des Wärmespeichers an die Unterseite des Körpers erfolgt typischerweise mittels eines Befestigungsmittels, beispielsweise einer Lötung, insbesondere einer Grafitlötung (z.B. Titan- oder Zirkonlötung). Der Wärmespeicher ist typischerweise rotationssymmetrisch um die Symmetrieachse des Körpers ausgebildet. Die wärmeleitende Kopplung der Unterseite des Körpers mit einem Wärmespeicher, der eine höhere Wärmeleitfähigkeit und/oder Wärmekapazität aufweist, ermöglicht eine effektive Wärmeverteilung und -speicherung, was Temperaturspitzen im Betrieb abmildern kann. Durch die Verwendung eines Wärmespeichers mit überlegenen thermischen Eigenschaften kann die thermische Stabilität des Geräts erhöht werden, was zu einer zuverlässigeren Funktion unter verschiedenen Betriebsbedingungen führt. Die verbesserte Wärmeableitung und -speicherung kann die Notwendigkeit einer zusätzlichen Kühlung reduzieren, was die Energieeffizienz des Geräts steigert und Betriebskosten senkt.

Die Kathode 611 kann insbesondere einen Elektronenemitter und/oder einen Fokuskopf 615 umfassen. Der Elektronenemitter kann insbesondere ein kalter Elektronenemitter oder ein warmer Elektronenemitter sein. Der Elektronenemitter ist beispielsweise eine Glühkathode oder eine Feldemissionskathode. Die Glühkathode erzeugt Elektronen durch thermische Emission, bei der die Elektronen durch Erhitzen aus dem Material gelöst werden. Dies wird als heiße Elektronenemission oder thermionische Emission bezeichnet. Die Feldemissionskathode erzeugt Elektronen durch den Feldeffekt, bei dem die Elektronen durch ein starkes elektrisches Feld aus den in Spitzen vorliegenden Material gezogen werden. Dies wird als kalte Elektronenemission bezeichnet. Die Kathode 611 ist vorzugsweise auf eine negativere Spannung gegenüber der Anode 612 geschaltet, um die Elektronen zu beschleunigen. Die Beschleunigungsspannung bestimmt die Energie und die Intensität der erzeugten Röntgenstrahlung R.

Das Vakuumgehäuse 610 ist insbesondere mit einem Hochvakuum evakuierbar oder evakuiert. Das Vakuumgehäuse 610 besteht beispielsweise aus Metall und/oder Glas. Zwischen der Kathode 611 und der Anode 612 ist vorzugsweise die Beschleunigungsspannung anlegbar. Insbesondere ein Hochspannungsgenerator kann die Beschleunigungsspannung bereitstellen.

Die Röntgeneinheit 601 ist über Lager in einem Eintank 602 angeordnet. Der Eintank 602 kann zur thermischen und/oder elektrischen Isolierung mit einem Öl gefüllt sein. Der Eintank 602 sowie die Lager, die im Eintank 602 ebenfalls angeordnete Elektronik, Kühlung etc. sind aus Gründen der Übersichtlichkeit nicht dargestellt. Aus dem Stand der Technik ist bekannt, wie derartige Standardbauteile angeordnet und ausgebildet sein können.

In dem dargestellten Ausführungsbeispiel ist eine Kollimatoreinheit 300 innerhalb des Eintanks angeordnet. Die Kollimatoreinheit 300 kann, insbesondere wie bezüglich einer der **Fig. 3a bis 3f** beschrieben oder eine Kombination, ausgebildet sein.

**FIG. 6b** zeigt ein zweites Ausführungsbeispiel einer Röntgenquelle 600 mit einer Kollimatoreinheit 300 vor dem Röntgenaustrittsfenster 614.

Die Röntgenquelle 600 ist grundsätzlich gemäß der Beschreibung zu **Fig. 6a** aufgebaut.

Die Kollimatoreinheit 300 kann, insbesondere wie bezüglich einer der **Fig. 3a bis 3f** beschrieben oder eine Kombination, ausgebildet sein.

Die Kollimatoreinheit 300 kann fest oder lösbar vor dem Röntgenaustrittsfenster 614 angeordnet sein.

**FIG. 6c** zeigt ein drittes Ausführungsbeispiel einer Röntgenquelle 600 mit einer Ablenkeinheit 617a.

Die Röntgenquelle 600 ist grundsätzlich gemäß der Beschreibung zu **Fig. 6a** aufgebaut.

Die Ablenkeinheit 617a ist dabei außerhalb des Vakuumgehäuses 610 aber innerhalb des Eintanks 602 angeordnet. Alternativ kann die Ablenkeinheit 617a auch außerhalb des Eintanks 602 angeordnet sein. Die Ablenkeinheit 617a kann dabei insbesondere um das Röntgenaustrittsfenster 614 angeordnet sein.

Die Ablenkeinheit 617a kann in einem Ausführungsbeispiel als Spule insbesondere als Luftspule ausgebildet sein.

Alternativ kann die Ablenkeinheit 617a als elektrostatische Ablenkeinheit ausgebildet sein.

In dem dritten Ausführungsbeispiel ist die Kollimatoreinheit 300, wie in Fig. 6b beschrieben dargestellt. Alternativ kann das dritte Ausführungsbeispiel auch mit anderen Ausführungsformen der Kollimatoreinheit 300 kombiniert werden, beispielsweise wie in Fig. 6a dargestellt oder wie in der Beschreibung beschrieben.

FIG. 6d zeigt ein viertes Ausführungsbeispiel einer Röntgenquelle 600 mit einer Ablenkeinheit 617b.

Die Röntgenquelle 600 ist grundsätzlich gemäß der Beschreibung zu Fig. 6a aufgebaut.

Die Ablenkeinheit 617b ist dabei innerhalb des Vakuumgehäuses 610 angeordnet.

In einem Ausführungsbeispiel kann die Ablenkeinheit 617b Teil des Fokuskopfes 615 sein.

Die Ablenkeinheit 617b kann den Elektronenstrahl mittels elektrischer oder magnetischer Ablenkung ablenken. Dafür kann die Ablenkeinheit 617b als Spule und/oder Ablenkplatten ausgebildet sein.

In Ausführungen der Erfindung kann eine Ablenkeinheit 617a außerhalb des Vakuumgehäuses 610 wie in **Fig. 6c** beschrieben mit einer Ablenkeinheit 617b im Vakuumgehäuse 610 wie in **Fig. 6d** beschrieben kombiniert werden.

In dem vierten Ausführungsbeispiel ist die Kollimatoreinheit 300, wie in **Fig. 6b** beschrieben dargestellt. Alternativ kann das vierte Ausführungsbeispiel auch mit anderen Ausführungsformen der Kollimatoreinheit 300 kombiniert werden, beispielsweise wie in **Fig. 6a** dargestellt oder wie in der Beschreibung beschrieben.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Computerimplementiertes Verfahren zum Beleuchten eines Röntgenstrahlungsfeldes (200) umfassend folgende Schritte:
- Bewegen (MOV-S) einer Röntgenquelle (600) relativ zu einem Röntgendetektor (505) während eines ersten und eines zweiten Belichtungszeitraums (T1, T2),
wobei der Röntgendetektor (505) dazu ausgebildet ist, von der Röntgenquelle (600) ausgesendete Röntgenstrahlung (R) während des ersten und des zweiten Belichtungszeitraums (T1, T2) zu empfangen (ACQ1, ACQ2),
wobei zwischen der Röntgenquelle (600) und dem Röntgendetektor (505) eine Kollimatoreinheit (300, 300', 300", 300‴, 300ʺʺ, 300‴ʺ) angeordnet ist,
wobei die Kollimatoreinheit (300, 300', 300", 300‴, 300"", 300‴ʺ) dazu ausgebildet ist, die Röntgenstrahlung (R) auf das Röntgenstrahlungsfeld (200) zu begrenzen,
- Bewegen (MOV-F1) eines Fokus' (630) der Röntgenstrahlung (R) relativ zu der Bewegung der Röntgenquelle (600) während des ersten Belichtungszeitraums (T1),
- Bewegen (MOV-C1) der Kollimatoreinheit (300, 300', 300", 300‴, 300"", 300‴ʺ) derart, dass während des ersten Belichtungszeitraums (T1) das Röntgenstrahlungsfeld (200) konstant beleuchtet wird,
- Bewegen (MOV-F2) des Fokus' (630) der Röntgenstrahlung (R) relativ zu der Bewegung der Röntgenquelle (600) während des zweiten Belichtungszeitraums (T2),
- Bewegen (MOV-C2) der Kollimatoreinheit (300, 300', 300", 300‴, 300"", 300‴ʺ) derart, dass während des zweiten Belichtungszeitraums (T2) das Röntgenstrahlungsfeld (200) konstant beleuchtet wird,
wobei die Kollimatoreinheit (300, 300', 300", 300‴, 300ʺʺ, 300‴ʺ) zu Beginn des ersten Belichtungszeitraums (T1) eine erste Startposition (SPOS1) einnimmt und zu Beginn des zweiten Belichtungszeitraums (T2) eine zweite Startposition (SPOS2) einnimmt,
wobei die erste und die zweite Startposition (SPOS1, SPOS2) verschieden sind.

2. Verfahren nach Anspruch 1,
wobei die Bewegung des Fokus' (630) während des ersten und des zweiten Belichtungszeitraums (T1, T2) derart ausgebildet ist, dass die Bewegung der Röntgenquelle (600) relativ zum Röntgendetektor (505) wenigstens teilweise derart kompensiert wird, dass der Fokus (630) aus Sicht des Röntgendetektors (505) ortsfest ist.

3. Verfahren nach Anspruch 2,
wobei die Kollimatoreinheit (300, 300', 300", 300‴, 300ʺʺ, 300‴ʺ) während des ersten und/oder des zweiten Belichtungszeitraums (T1, T2) entgegengesetzt zu der Bewegung der Röntgenquelle (600) bewegt wird.

4. Verfahren nach einem der vorhergehenden Ansprüche,
wobei das Röntgenstrahlungsfeld (200) auf einer Oberfläche (505.1) des Röntgendetektors (500) angeordnet ist.

5. Verfahren nach einem der Ansprüche 1 bis 3,
wobei zwischen der Röntgenquelle (600) und dem Röntgendetektor (500) ein Untersuchungsobjekt (O) angeordnet ist,
wobei das Röntgenstrahlungsfeld (200) in einer Ebene innerhalb des Untersuchungsobjektes (O) angeordnet ist.

6. Verfahren nach einem der vorhergehenden Ansprüche,
wobei die Kollimatoreinheit (300, 300', 300", 300‴, 300"", 300‴ʺ) basierend auf der Bewegung des Fokus' (630) bewegt wird.

7. Verfahren nach Anspruch 6,
wobei die Kollimatoreinheit (300, 300', 300", 300‴, 300"", 300‴ʺ) außerdem basierend auf der Bewegung der Röntgenquelle (600) bewegt wird.

8. Verfahren nach einem der vorhergehenden Ansprüche,
wobei die erste Startposition (SPOS1) der Kollimatoreinheit (300, 300', 300", 300"', 300ʺʺ, 300‴ʺ) von einer Position der Röntgenquelle (600) relativ zum Röntgendetektor (505) zu Beginn des ersten Belichtungszeitraums (T1) abhängt,
wobei die zweite Startposition (SPOS2) der Kollimatoreinheit (300, 300', 300", 300"', 300"", 300‴ʺ) von einer Position der Röntgenquelle (600) relativ zum Röntgendetektor (505) zu Beginn des zweiten Belichtungszeitraums (T2) abhängt.

9. Verfahren nach einem der vorhergehenden Ansprüche,
wobei die Kollimatoreinheit (300, 300', 300", 300‴, 300"", 300‴ʺ) wenigstens zwei Lamellen (301.1, 301.1ʺʺ, 301.2, 301.2', 301.2"", 301.3, 301.3", 301.3"', 301.3ʺʺ, 301.4, 304.4", 301.4‴, 301.4"", 301a, 301b, 301c, 301d) umfasst,
wobei die erste Startposition (SPOS1) wenigstens eine Startposition wenigstens einer der Lamellen (301.1, ..., 301d) relativ zu der Röntgenquelle (600) zu Beginn des ersten Belichtungszeitraums (T1) vorgibt,
wobei die zweite Startposition (SPOS2) wenigstens eine Startposition wenigstens einer der Lamellen (301.1, ..., 301d) relativ zu der Röntgenquelle (600) zu Beginn des zweiten Belichtungszeitraums (T2) vorgibt.

10. Verfahren nach einem der vorhergehenden Ansprüche,
wobei die Kollimatoreinheit (300, 300', 300", 300‴, 300"", 300‴ʺ) wenigstens zwei Lamellen (301.1, ..., 301d) umfasst,
wobei die erste Startposition (SPOS1) wenigstens einen Abstand der beiden Lamellen (301.1, ..., 301d) zueinander zu Beginn des ersten Belichtungszeitraums (T1) vorgibt,
wobei die zweite Startposition (SPOS2) wenigstens einen Abstand der beiden Lamellen (301.1, ..., 301d) zueinander zu Beginn des zweiten Belichtungszeitraums (T2) vorgibt.

11. Verfahren nach einem der vorhergehenden Ansprüche,
wobei die Bewegung der Röntgenquelle (600) kontinuierlich ist,
insbesondere wobei die Bewegung der Röntgenquelle (600) als eine bogenförmige Bewegung relativ zu dem Röntgendetektor (505) ausgebildet ist.

12. Kollimatoreinheit (300, 300', 300", 300"', 300ʺʺ, 300‴ʺ) ausgebildet zur Verwendung in einem Verfahren nach einem der Ansprüche 1 bis 11.

13. Kollimatoreinheit (300, 300', 300", 300‴, 300"", 300‴ʺ) nach Anspruch 12,
wobei die Kollimatoreinheit (300, 300', 300", 300‴, 300"", 300‴ʺ) wenigstens zwei Lamellen (301.1, ..., 301d) umfasst.

14. Kollimatoreinheit (300, 300', 300", 300"', 300ʺʺ, 300‴ʺ) nach Anspruch 13,
wobei die wenigstens zwei Lamellen (301.1, ..., 301d) unabhängig voneinander bewegbar sind.

15. Kollimatoreinheit (300, 300', 300", 300‴, 300"", 300‴ʺ) nach einem der Ansprüche 13 oder 14,
wobei jede der Lamellen (301.1, ..., 301d) an einer Linearverstelleinheit angeordnet ist.

16. Kollimatoreinheit (300, 300', 300", 300"', 300ʺʺ, 300‴ʺ) nach einem der Ansprüche 13 bis 15,
wobei wenigstens eine der Lamellen (301.1, ..., 301d) rotierbar ausgebildet ist.

17. Kollimatoreinheit (300, 300', 300", 300‴, 300ʺʺ, 300‴ʺ) nach einem der Ansprüche 13 bis 16,
wobei wenigstens eine der Lamellen (301.1, ..., 301d) eine gekrümmte Kante aufweist.

18. Kollimatoreinheit (300, 300', 300", 300‴, 300"", 300‴ʺ) nach einem der Ansprüche 13 bis 17,
wobei wenigstens eine der Lamellen (301.1, ..., 301d) eine variable Dicke aufweist.

19. Kollimatoreinheit (300, 300', 300", 300‴, 300"", 300‴ʺ) nach Anspruch 18,
wobei die Dicke der wenigstens einen Lamelle (301.1, ..., 301d) derart ausgebildet ist, dass eine räumliche Inhomogenität der ausgesendeten Röntgenstrahlung (R) am Röntgendetektor (505) kompensiert wird.

20. Steuersystem (SYS),
umfassend eine Recheneinheit (SYS.CU) und eine Schnittstelle (SYS.IF),
wobei die Recheneinheit (SYS.CU) und die Schnittstelle (SYS.IF) dazu ausgebildet sind, alle Schritte des Verfahrens nach einem der Ansprüche 1 bis 11 auszuführen.

21. Mammographiesystem (500) umfassend:
- eine Röntgenquelle (600),
- eine Kollimatoreinheit (300, 300', 300", 300‴, 300"", 300‴ʺ) nach einem der Ansprüche 12 bis 19,
- eine Kompressionseinheit (501),
- einen Röntgendetektor (505) und
- ein Steuersystem (SYS) gemäß Anspruch 20.

22. Mammographiesystem (500) nach Anspruch 21,
wobei die Röntgenquelle (600) relativ zu dem Röntgendetektor (505) beweglich ausgebildet ist.

23. Mammographiesystem (500) nach Anspruch 22,
wobei die Röntgenquelle (600) um mehr als 30° relativ zu dem Röntgendetektor (505) beweglich ist.

24. Mammographiesystem (500) nach einem der Ansprüche 21 bis 23,
wobei die Röntgenquelle (600) dazu ausgebildet ist, einen Fokus (630) der von der Röntgenquelle (600) ausgesendeten Röntgenstrahlung (R) zu bewegen.

25. Mammographiesystem (500) nach einem der Ansprüche 21 bis 24,
wobei die Kollimatoreinheit (300, 300', 300", 300‴, 300ʺʺ, 300‴ʺ) auf einer Achse zwischen Röntgenquelle (600) und Röntgendetektor (505) verfahrbar ausgebildet ist.

26. Mammographiesystem (500) nach einem der Ansprüche 21 bis 25,
wobei die Kollimatoreinheit (300, 300', 300", 300‴, 300"", 300‴ʺ) in einem Eintank (602) der Röntgenquelle (600) angeordnet ist.

27. Mammographiesystem (500) nach einem der Ansprüche 21 bis 25,
wobei die Kollimatoreinheit (300, 300', 300", 300‴, 300ʺʺ, 300‴ʺ) vor einem Röntgenaustrittsfenster (614) der Röntgenquelle (600) angeordnet ist.

28. Mammographiesystem (500) nach einem der Ansprüche 21 bis 25,
wobei die Kompressionseinheit (501) wenigstens eine Kompressionsplatte (506) umfasst,
wobei die Kollimatoreinheit (300, 300', 300", 300‴, 300ʺʺ, 300‴ʺ) auf der Kompressionsplatte (506) oder auf dem Röntgendetektor (505) angeordnet ist.

29. Mammographiesystem (500) nach einem der Ansprüche 21 bis 28,
wobei die Kollimatoreinheit (300, 300', 300", 300‴, 300"", 300‴ʺ) wenigstens eine erste und eine zweite Sub-Einheit umfasst,
wobei die erste Sub-Einheit in einem ersten Abstand zu dem Röntgendetektor (505) angeordnet ist,
wobei die zweite Sub-Einheit in einem zweiten Abstand zu dem Röntgendetektor (505) angeordnet ist,
wobei der erste und der zweite Abstand verschieden ist.

30. Computerprogrammprodukt mit einem Computerprogramm, welches direkt in einen Speicher eines Steuersystems (SYS) ladbar ist, mit Programmabschnitten, um alle Schritte des Verfahrens nach einem der Ansprüche 1 bis 11 auszuführen, wenn die Programmabschnitte von dem Steuersystem (SYS) ausgeführt werden.

31. Computerlesbares Speichermedium, auf welchem von einem Steuersystem (SYS) lesbare und ausführbare Programmabschnitte gespeichert sind, um alle Schritte des Verfahrens nach einem der Ansprüche 1 bis 11 auszuführen, wenn die Programmabschnitte von dem Steuersystem (SYS) ausgeführt werden.
